# EUROPEAN PATENT SPECIFICATION

(11) **EP 3 928 862 B1**
(45) Date of publication and mention of the grant of the patent: **03.06.2026**
(21) Application number: 20786705.2
(22) Date of filing: 02.03.2020
(51) Int. Cl.: G16C 60/00, C07K 5/062, C07K 5/083, G01N 15/0227, A01N 1/125, G01N 33/18

(54) **BIOMIMETIC ICE-INHIBITING MATERIAL AND CRYOPRESERVATION LIQUID CONTAINING SAME**
BIOMIMETISCHES EISHEMMENDES MATERIAL UND DIESES ENTHALTENDE KRYOKONSERVIERUNGSFLÜSSIGKEIT
MATÉRIAU BIOMIMÉTIQUE INHIBANT LA GLACE ET LIQUIDE DE CRYOCONSERVATION LE CONTENANT

(30) Priority: 09.04.2019 CN 201910282418; 09.04.2019 CN 201910282422; 09.04.2019 CN 201910282417; 09.04.2019 CN 201910282416; 09.04.2019 CN 201910281986
(43) Date of publication of application: 29.12.2021
(73) Proprietor: INSTITUTE OF CHEMISTRY, CHINESE ACADEMY OF SCIENCES, Beijing 100190 (CN); Peking University Third Hospital, Beijing 100191 (CN)
(72) Inventor: WANG, Jianjun, Beijing 100190 (CN); JIN, Shenglin, Beijing 100190 (CN); LV, Jianyong, Beijing 100190 (CN); YAN, Jie, Beijing 100191 (CN); QIAO, Jie, Beijing 100191 (CN); YAN, Liying, Beijing 100191 (CN); LI, Rong, Beijing 100191 (CN)
(74) Representative: Gille Hrabal Partnerschaftsgesellschaft mbB Patentanwälte
(86) International application number: PCT/CN2020/077472
(87) International publication number: WO 2020/207150

(56) References cited:
- WO-A1-2013/117925
- WO-A1-91/10361
- WO-A2-2017/066454
- CN-A- 101 622 986
- CN-A- 102 726 366
- CN-A- 104 839 144
- CN-A- 106 544 385
- CN-A- 107 183 008
- CN-A- 107 183 008
- CN-A- 108 207 930
- CN-A- 109 221 082
- CN-A- 109 497 044
- US-A1- 2010 068 692
- WENG LINDONG ET AL: "Molecular Dynamics at the Interface between Ice and Poly(vinyl alcohol) and Ice Recrystallization Inhibition", LANGMUIR, vol. 34, no. 17, 4 December 2017 (2017-12-04), US, pages 5116 - 5123, XP093034891, ISSN: 0743-7463, DOI: 10.1021/acs.langmuir.7b03243
- DRORI RAN ET AL: "A Supramolecular Ice Growth Inhibitor", JOURNAL OF THE AMERICAN CHEMICAL SOCIETY, vol. 138, no. 40, 12 September 2016 (2016-09-12), pages 13396 - 13401, XP093034878, ISSN: 0002-7863, DOI: 10.1021/jacs.6b08267
- DRORI RAN ET AL: "Supplementary Information to A Supramolecular Ice Growth Inhibitor", 12 September 2016 (2016-09-12), XP093034946, Retrieved from the Internet <URL:https://pubs.acs.org/doi/10.1021/jacs.6b08267#article_content-right> [retrieved on 20230327]
- JIN SHENGLIN ET AL: "Spreading fully at the ice-water interface is required for high ice recrystallization inhibition activity", SCIENCE CHINA CHEMISTRY; THE FRONTIERS OF CHEMICAL BIOLOGY AND SYNTHESIS, SCIENCE CHINA PRESS, SIENCE CHINA PRESS, vol. 62, no. 7, 6 May 2019 (2019-05-06), pages 909 - 915, XP036816484, ISSN: 1674-7291, [retrieved on 20190506], DOI: 10.1007/S11426-018-9428-4
- MUHAMMAD HASAN ET AL: "Ice Recrystallization Inhibiting Polymers Enable Glycerol-Free Cryopreservation of Microorganisms", BIOMACROMOLECULES, vol. 19, no. 8, 22 June 2018 (2018-06-22), US, pages 3371 - 3376, XP055587871, ISSN: 1525-7797, DOI: 10.1021/acs.biomac.8b00660
- MOCHIZUKI KENJI ET AL: "Antifreeze Glycoproteins Bind Reversibly to Ice via Hydrophobic Groups", vol. 140, no. 14, 2 February 2018 (2018-02-02), pages 4803 - 4811, XP093064580, ISSN: 0002-7863, Retrieved from the Internet <URL:https://pubs.acs.org/doi/pdf/10.1021/jacs.7b13630> DOI: 10.1021/jacs.7b13630
- BEN GRAHAM ET AL: "Polyproline as a Minimal Antifreeze Protein Mimic That Enhances the Cryopreservation of Cell Monolayers", ANGEWANDTE CHEMIE INTERNATIONAL EDITION, VERLAG CHEMIE, HOBOKEN, USA, vol. 56, no. 50, 22 November 2017 (2017-11-22), pages 15941 - 15944, XP072091811, ISSN: 1433-7851, DOI: 10.1002/ANIE.201706703
- LUUK L. C. OLIJVE ET AL: "Influence of Polymer Chain Architecture of Poly(vinyl alcohol) on the Inhibition of Ice Recrystallization", MACROMOLECULAR CHEMISTRY AND PHYSICS, vol. 217, no. 8, 1 April 2016 (2016-04-01), DE, pages 951 - 958, XP055621429, ISSN: 1022-1352, DOI: 10.1002/macp.201500497
- STUBBS CHRISTOPHER ET AL: "Photo-polymerisation and study of the ice recrystallisation inhibition of hydrophobically modified poly(vinyl pyrrolidone) co-polymers", EUROPEAN POLYMER JOURNAL, vol. 110, 30 November 2018 (2018-11-30), pages 330 - 336, XP085572046, ISSN: 0014-3057, DOI: 10.1016/J.EURPOLYMJ.2018.11.047
- JIN SHENGLIN ET AL: "Supporting Information Spreading fully at the ice-water interface is required for high ice recrystallization inhibition activity", 6 May 2019 (2019-05-06), XP093058927, Retrieved from the Internet <URL:https://static-content.springer.com/esm/art%3A10.1007%2Fs11426-018-9428-4/MediaObjects/11426_2018_9428_MOESM1_ESM.pdf> [retrieved on 20230628]
- 薛涵 等 (XUE, HAN ET AL.): "亲水性高分子对冰重结晶的抑制作用研究 (Ice Recrystallisation Inhibition by Hydrophilic Polymers)", 高分子通报 (POLYMER BULLETIN), no. 9, 30 September 2016 (2016-09-30), XP55742565, DOI: 20200518083919
- 吕健勇 等 (LV, JIANYONG ET AL.): "防冰高分子材料 (Recent Research on Polymeric Anti-icing Materials)", 高分子学报 (ACTA POLYMERICA SINICA), no. 12, 31 December 2017 (2017-12-31), XP55742567, DOI: 20200518084257A

## Description

The present application claims priority to Chinese Patent Nos. 2019102824189, 2019102824225, 2019102824174, 201910282416X and 2019102819867 filed with the China National Intellectual Property Administration on April 9, 2019.

### TECHNICAL FIELD

The present invention relates to the technical field of biomedical materials, and particularly to a biomimetic ice growth inhibition material and a cryopreservation solution comprising the same.

### BACKGROUND

Cryopreservation is to store a biological material at an ultra-low temperature to slow down or stop cell metabolism and division, and to continue development once normal physiological temperature is recovered. Since its advent, this technology has become one of indispensable research methods in the field of natural science, and has been widely adopted. In recent years, with the increasing pressure of life, human fertility tends to decline year by year, and thus fertility preservation is more and more emphasized. The cryopreservation of human germ cells (sperms and oocytes), gonad tissues and the like has become an important means of fertility preservation. In addition, as the world population ages, the need for cryopreservation of donated human cells, tissues or organs that can be used for regenerative medicine and organ transplantation is growing fast. Therefore, how to efficiently cryopreserve precious cells, tissues and organ resources for unexpected needs has become a scientific and technical problem to be solved urgently. Currently, the most commonly available cryopreservation method is vitrification. The vitrification technology adopts a permeable or impermeable cryoprotectant. Although liquid inside and outside a cell can be directly vitrified in the rapid freezing process so as to avoid the damage resulting from the formation of ice crystals in the freezing process, cryopreservation reagents of the prior art are not effective in controlling the growth of the ice crystals in the rewarming process and thus damage the cell. Because the ice growth inhibition mechanisms of antifreeze proteins and biomimetic ice growth inhibition materials at the molecular level is still controversial, the research and development of the biomimetic ice growth inhibition materials has to rely on "trial and error" to test the ice growth inhibition effect of a certain ice growth inhibition material, which is characterized by a heavy workload, and a low probability of success. Currently, commonly available cryopreservation reagents have the problems of having no capability of effectively controlling the growth of ice crystals in the rewarming process and being high in toxicity. Weng Lingdong et al. ("Molecular Dynamics at the Interface between Ice and Poly(vinyl alcohol) and Ice Recrystallization Inhibition", Langmuir, vol. 34, no. 17, 4.12.2017, pp. 5116-5123) discloses molecular dynamics of ice recrystallization inhibition by poly(vinyl alcohol).

### SUMMARY

In order to overcome the defects in the prior art, the present invention provides a molecular design method for a biomimetic ice growth inhibition material and a screening method for an ice growth inhibition material, which can guide people to purposefully synthesize and screen the biomimetic ice growth inhibition material. The present invention also provides a biomimetic ice growth inhibition material obtained based on the method and a cryopreservation reagent comprising the same.

The present invention provides the following technical solutions:
In a first aspect of the present invention, a molecular design method for an ice growth inhibition material is provided, comprising the following steps:
(1) constructing a library for structures of compound molecules, wherein the compound molecules comprise a hydrophilic group and an ice-philic group;
(2) simulating and evaluating the spreading performance of each of the compound molecules at an ice-water interface by adopting molecular dynamics (MD) simulation; and
(3) screening the ice growth inhibition molecules with desired affinities for ice and water according to the step (2).

According to the present invention, the main chain of the ice growth inhibition molecule is a carbon chain or a peptide chain.

According to the present invention, the hydrophilic group is a functional group capable of forming a non-covalent interaction with a water molecule, for example, forming a hydrogen bond, a Van der Waals interaction, an electrostatic interaction, a hydrophobic interaction or a π-π interaction with water; illustratively, the hydrophilic group may be selected from at least one of hydroxyl (-OH), amino (-NH₂), carboxyl (-COOH), amido (-CONH₂), and the like, or, for example, from a compound molecule, such as a hydrophilic amino acid such as proline (L-Pro), arginine (L-Arg) and lysine (L-Lys), glucono delta-lactone (GDL) and a saccharide, and a molecular fragment thereof.

According to the present invention, the ice-philic group is a functional group capable of forming a non-covalent interaction with ice, for example, forming a hydrogen bond, a Van der Waals interaction, an electrostatic interaction, a hydrophobic interaction or a π-π interaction with ice; illustratively, the ice-philic group may be selected from hydroxyl (-OH), amino (-NH₂), phenyl (-C₆H₅) and pyrrolidinyl (-C₄H₈N), or, for example, from a compound molecule, such as an ice-philic amino acid such as glutamine (L-Gln), threonine (L-Thr) and aspartic acid (L-Asn), a benzene ring (C₆H₆) and pyrrolidine (C₄H₉N), and a molecular fragment thereof.

According to the present invention, the ice growth inhibition material may be formed by bonding a hydrophilic group and an ice-philic group through a covalent bond.

According to the present invention, the ice growth inhibition material may be formed from a hydrophilic group and an ice-philic group through a non-covalent bond such as an ionic interaction.

In the present invention, "cryopreservation" and "cryogenic preservation" have the same meaning and are used interchangeably, and refer to preservation of a substance, or a cell, a tissue, or an organ at a low temperature to retain the original physicochemical and/or biological activity, and physiological and biochemical functions thereof.

In the present invention, the term "ice growth inhibition molecule" or "ice growth inhibition material" has the same meaning and refers to a compound capable of inhibiting the growth of ice crystals in an aqueous solution. In one embodiment, the ice growth inhibition molecule has good spreading performance at an ice-water interface and can reduce the grain size of ice crystals, or the ice growth inhibition molecule has no thermal hysteresis or has sufficiently small thermal hysteresis to significantly reduce ice crystal formation in an aqueous solution.

### Beneficial Effects

1. In the present invention, a mechanism of controlling the growth of ice crystals in an ice-water mixed phase by an ice growth inhibition molecule is found for the first time. The ice growth inhibition material is required to have good affinities for both ice and water. The affinity of the ice growth inhibition molecules for ice can ensure that the ice growth inhibition molecules are well adsorbed on the surface of the ice; the affinity of the molecules for water can ensure that the molecules better spread on an ice-water interface to cover the maximum ice surface area with as less amount of material as possible. Based on the ice growth inhibition mechanism, an idea of designing an ice growth inhibition molecule with affinities for both ice and water is proposed, which provides a new method for synthesizing an ice growth inhibition material.
2. In the present invention, MD simulation is introduced into the design of the molecular structure of an ice growth inhibition material for the first time, through which the affinities of the designed ice growth inhibition molecule for ice and water are evaluated, the ice growth inhibition performance of the ice growth inhibition material is predicted and thus optimization of the structure can be achieved.
3. In the present invention, the combination of the ice growth inhibition mechanism and MD simulation well solves the limitation that the materials known in the art can be subjected to performance analysis and screening only by the experimental "trial and error" approach in the research and development process of an ice growth inhibition material, provides a new idea of the design of a molecular structure and can significantly promote the development and application of the ice control material.
4. The cryopreservation solution provided (but not claimed) herein has the advantages of wide sources, good biocompatibility, low toxicity, high safety and a greatly reduced amount of DMSO used, and can achieve an equal or even higher cell survival rate than that of a commercial cryopreservation solution comprising DMSO of no less than 15% and commonly available in clinical practice at present when used even without the addition of DMSO. The cryopreservation solution disclosed herein has advantages of simple composition, readily available starting materials and low costs, and can be widely applied to cryopreservation of various cells and tissues, such as oocytes, embryos, stem cells, ovarian tissues and ovarian organs, to retain good biological activity.

### BRIEF DESCRIPTION OF THE DRAWINGS

FIG. 1: a schematic diagram of the molecular structure of the ice growth inhibition material;
FIG. 2: aggregation states of an atactic PVA (a-PVA) and an isotactic PVA (i-PVA) at an ice-water interface by MD simulation;
FIG. 3: a proton nuclear magnetic resonance (NMR) spectrum of the a-PVA synthesized in Example 1;
FIG. 4: proton NMR spectra of the PBVE and the i-PVA synthesized in Example 1, wherein A is for the PBVE and B is for the i-PVA;
FIG. 5: GPC curves of the PBVE synthesized in Example 1;
FIG. 6: dispersion sizes of the a-PVA (A) and the i-PVA (B) in water at different concentrations in the DLS experiment;
FIG. 7: micrographs of ice crystal growth in solutions of the two PVAs, wherein A is for the a-PVA and B is for the i-PVA; C shows the relationship of the two PVAs between the mean largest grain size and the concentration relative to that of PBS;
FIG. 8: the topography of ice crystals modified by the a-PVA (FIGs. A and B) and i-PVA (FIGs. C and D) in purified water;
FIG. 9: molecular structure models of the two PVAs by MD simulation;
FIG. 10: contactable surface areas of the two PVA molecules with water molecules and ice-water molecules at an ice-water interface at 240 K by MD simulation, wherein the upper part of the figure shows 3 results of the a-PVA molecules, and the lower part of the figure shows 3 results of the i-PVA molecules;
FIG. 11: aggregation probabilities of the two PVAs in an aqueous solution by MD simulation and calculations;
FIG. 12: the number of the intermolecular hydrogen bonds formed between the two PVAs and water at 240 K in an aqueous solution, and the number of the intermolecular hydrogen bonds formed between the two PVAs and water molecules and ice-water molecules at 240 K at an ice-water interface by MD simulation and calculations;
FIG. 13: optical micrographs of the inhibition of the growth activity of ice crystals by GDL-L-Thr (compound of formula (6)) and a statistical diagram of grain size;
FIG. 14: the topography of an ice crystal modified by GDL-L-Thr in purified water;
FIG. 15: optical micrographs of the inhibition of the growth activity of ice crystals by GDL-L-Ser (compound of formula (7)) and a statistical diagram of grain size;
FIG. 16: optical micrographs of the inhibition of the growth activity of ice crystals by GDL-L-Val (compound of formula (8)) and a statistical diagram of grain size;
FIG. 17: optical micrographs of the inhibition of the growth activity of ice crystals by the TR short-chain peptide prepared in Example 3 and a statistical diagram of grain size;
FIG. 18: the topography of an ice crystal modified by the TR short-chain peptide prepared in Example 3 in purified water;
FIG. 19: inhibition results of the growth activity of ice crystals by the peptoids R-COOH, R-CH₃ and R-CH₂CH₃ in Example 8;
FIG. 20: the topographies of ice crystals modified by the peptoids R-COOH (A), R-CH₃ (B) and R-CH₂CH₃ (C) in Example 8 in purified water;
FIG. 21: a schematic diagram of an ice adsorption experiment and a device thereof;
FIG. 22: diagrams of the ice adsorption amount vs. the concentration for the two PVAs in Example 9;
FIG. 23: micrographs of ice crystal growth in DPBS solutions of the two PVAs, wherein A is for the a-PVA and B is for the i-PVA;
FIG. 24: a picture of a stained slice of a fresh (unfrozen) ovarian organ of a 3-day-old newborn mouse;
FIG. 25: a picture of a stained slice of the cryopreserved ovarian organ in Comparative Embodiment 8 after thawing;
FIG. 26: a picture of a stained slice of the cryopreserved ovarian organ in Application Embodiment 13 after thawing;
FIG. 27: a picture of a stained slice of the cryopreserved ovarian organ in Application Embodiment 14 after thawing;
FIG. 28: a picture of a stained slice of the cryopreserved ovarian organ in Application Embodiment 15 after thawing;
FIG. 29: a picture of a stained slice of fresh (unfrozen) ovarian tissue of a sexually mature mouse;
FIG. 30: a picture of a stained slice of the cryopreserved ovarian tissue in Comparative Embodiment 9 after thawing;
FIG. 31: a picture of a stained slice of the cryopreserved ovarian tissue in Application Embodiment 16 after thawing;
FIG. 32: a picture of a stained slice of the cryopreserved ovarian tissue in Application Embodiment 17 after thawing;
FIG. 33: a picture of a stained slice of the cryopreserved ovarian tissue in Application Embodiment 18 after thawing;
FIG. 34: a picture of a stained slice of the cryopreserved ovarian tissue in Application Embodiment 26 after thawing;

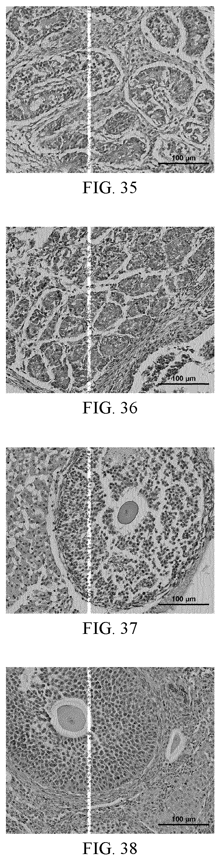

FIG. 35: a picture of a stained slice of the cryopreserved ovarian tissue in Application Embodiment 27 after thawing;
FIG. 36: a picture of a stained slice of the cryopreserved ovarian tissue in Application Embodiment 28 after thawing;
FIG. 37: a picture of a stained slice of the cryopreserved ovarian tissue in Application Embodiment 29 after thawing;
FIG. 38: a picture of a stained slice of the cryopreserved ovarian tissue in Application Embodiment 30 after thawing;
FIG. 39: a picture of a stained slice of the cryopreserved ovarian tissue in Application Embodiment 31 after thawing;
FIG. 40: a picture of a stained slice of the cryopreserved ovarian tissue in Application Embodiment 37 after thawing; and
FIG. 41: a picture of a stained slice of the cryopreserved ovarian tissue in Application Embodiment 38 after thawing.

### DETAILED DESCRIPTION

A preparation method (not claimed) will be further illustrated in detail with reference to the following specific examples. It should be understood that the following examples are merely exemplary illustration and explanation of the present invention, and should not be construed as limiting the protection scope of the present invention. Unless otherwise stated, the experimental methods used in the following examples are conventional methods. Unless otherwise stated, the reagents, materials, and the like used in the following examples are commercially available.

### A. Molecular design of ice growth inhibition material

The core molecule of the ice growth inhibition material disclosed herein can be designed to various groups having an affinity for water and groups having an affinity for ice, which are linked by covalent bonds or non-covalent bonds such as ionic bonds.

The molecular design method for the ice growth inhibition material disclosed herein comprises the following steps:
(1) constructing a library for structures of compound molecules, wherein the compound molecules comprise a hydrophilic group and an ice-philic group;
(2) simulating and evaluating the spreading performance of each of the compound molecules at an ice-water interface by adopting molecular dynamics (MD) simulation; and
(3) screening the ice growth inhibition molecule with desired affinities for ice and water.

According to the present invention, the main chain of the ice growth inhibition molecule is a carbon chain or a peptide chain.

According to the present invention, the MD simulation of the step (2) can be performed by GROMACS, AMBER, CHARMM, NAMD, or LAMMPS.

According to the present invention, in the MD simulation of the step (2), a model of a water molecule may be selected from models of TIP3P, TIP4P, TIP4P/2005, SPC, TIP3P, TIP5P, and SPC/E, preferably TIP4P/2005 model of a water molecule.

According to the present invention, in the MD simulation of the step (2), a force field parameter is provided by one of GROMOS, ESFF, MM force field, AMBER, CHARMM, COMPASS, UFF, CVFF and other force fields.

According to the present invention, in the MD simulation of the step (2), simulation and calculation are performed on interactions between ice growth inhibition molecules, interactions between ice growth inhibition molecules and water molecules, and interactions between ice growth inhibition molecules and ice-water molecules. The interactions include the formation of a hydrogen bond, a Van der Waals interaction, an electrostatic interaction, a hydrophobic interaction, a π-π interaction and the like.

According to the present invention, in the MD simulation of the step (2), when the simulated and calculated molecules interact, the temperature and pressure are adjusted. In one embodiment of the present invention, a V-rescale (modified Berendsen) temperature-regulator and a pressure-regulator are used to regulate the temperature and pressure.

According to the present invention, in the MD simulation of the step (2), the molecular configuration of the compound molecule is maintained by selecting a potential energy parameter. Preferably, the potential energy parameter is selected, so that the molecular configuration of the compound molecule is maintained at a higher temperature.

According to the present invention, in the step (2), periodic boundary conditions are adopted for x-direction, y-direction and z-direction when an aqueous solution system is simulated; periodic boundary conditions are adopted for x-direction and y-direction when an ice-water mixed system is simulated.

According to the present invention, in the MD simulation of the step (2), a cubic or octahedral box of water is selected, and a cubic box of water with dimensions of 3.9 × 3.6 × 1.0 nm³ is preferred.

As a specific embodiment of the present invention, during the process of molecular dynamics calculations of the MD simulation, the V-rescale (modified Berendsen) temperature-regulator and the pressure-regulator regulate the temperature and pressure.

In the MD simulation and calculations, the main criterion for determining the existence of a hydrogen bond is the energy criteria or the geometric criteria, preferably, the geometrical criteria; when the distance (pitch) between oxygen atoms is less than 0.35 nm and the angle HO...H is less than 30 degrees, a hydrogen bond between two hydroxyl groups or between a hydroxyl group and a water molecule is formed.

As a specific embodiment of the present invention, the ice growth inhibition material may be a compound that has a carbon chain as the main chain and is substituted with an ice-philic group and a hydrophilic group; the ice growth inhibition material may comprise a group that is both hydrophilic and ice-philic, such as hydroxyl and amino groups, and may further comprise an ice-philic group and a hydrophilic group separately. For example, the molecular structure of the ice growth inhibition material is designed to have a repeating unit -[CH₂-CHOH]-.

In an embodiment of the present invention, the molecule of the ice growth inhibition material is a PVA. The PVA is selected from one of or a combination of two or more of an isotactic PVA, a syndiotactic PVA and an atactic PVA. For example, the PVA has a diad syndiotacticity of 15%-65%, specifically, for example, 40%-60% and 53%55%. Atactic PVA is preferred, for example, the PVA with a diad syndiotacticity of 45%-65%. The PVA may be selected from a PVA having a molecular weight of 10-500 kDa or higher, such as 10-30 kDa, 30-50 kDa, 80-90 kDa, and 200-500 kDa. The PVA may be selected from a PVA having a degree of hydrolysis of greater than 80%, for example, 80%-99%, 82%-87%, 87%-89%, 89%-99%, and 98%-99%.

In an embodiment of the present invention, the molecule of the ice growth inhibition material is a peptidic compound. The peptidic compounds are obtained by reacting ice-philic amino acids, such as threonine (L-Thr), glutamine (L-Gln) and aspartic acid (L-Asn), with other hydrophilic amino acids that may be selected from arginine, proline, alanine, and the like, or GDL or saccharides.

The peptidic compound consists of no less than two amino acid units, such as: 2-8 amino acid units, specifically, 2-5, such as 2, 3, 4, 5 and 6 amino acid units; each amino acid unit is different. In the peptidic compound, the molar ratio of the ice-philic amino acid such as threonine to other hydrophilic amino acids is (0.1-3):1, preferably (0.5-2):1. The arrangement of the ice-philic amino acid and other hydrophilic amino acids in the peptidic compound is not particularly limited, and may be linked by using the amino acid linking groups or chemical bonds known in the art. For example, the ice-philic amino acid and the hydrophilic amino acid may be alternately arranged, or multiple ice-philic amino acids or hydrophilic amino acids are linked to form a fragment of the ice-philic amino acid or the hydrophilic amino acid, which is then linked to the hydrophilic amino acid (or a fragment) or the ice-philic amino acid (or a fragment), respectively.

In an embodiment of the present invention, the peptidic compound is at least one of L-Thr-L-Arg (TR), L-Thr-L-Pro (TP), L-Arg-L-Thr (RT), L-Pro-L-Thr (PT), L-Thr-L-Arg-L-Thr (TRT), L-Thr-L-Pro-L-Thr (TPT), L-Ala-L-Ala-L-Thr (AAT) and L-Thr-L-Cys-L-Thr (TCT).

In another embodiment, the peptidic compound is a GDL-L-amino acid, such as GDL-L-Thr, GDL-L-Ser or GDL-L-Val.

In yet another embodiment, the peptidic compound has any one of the structures of formula (1) to formula (8):

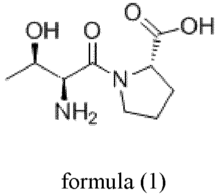

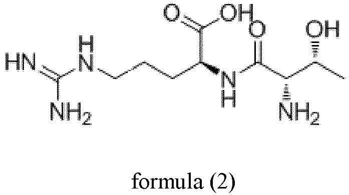

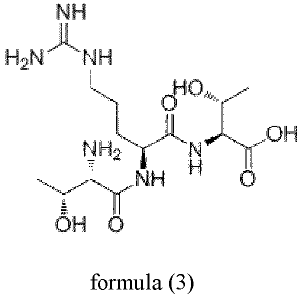

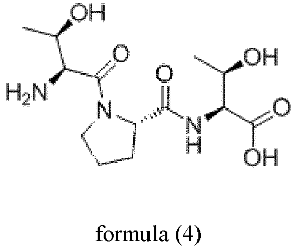

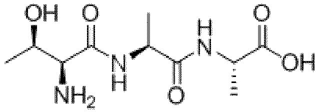

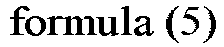

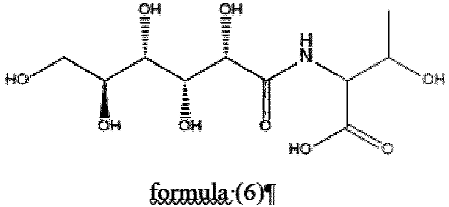

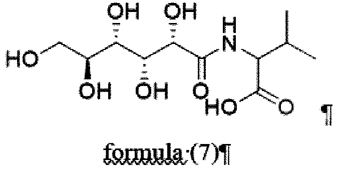

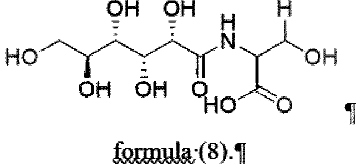

The above-mentioned peptidic compounds can be synthesized using a polypeptide synthesis method known in the art, such as a solid-phase synthesis method.

The preparation method disclosed but not claimed herein comprises the following steps: resin swelling, covalently linking an amino acid whose the amino group is protected to the swollen resin, deprotecting, adding another amino acid whose the amino group is protected for condensation reaction, deprotecting, cleavaging and purifying.

The glycopeptide derivative (not claimed) can be prepared by using the known method for reacting an amino acid with a saccharide. For example, the glycopeptide derivative can be prepared by reacting glucono delta-lactone or other saccharides with an amino acid in an organic solvent, or by using a solid-phase synthesis method. Glucono delta-lactone (GDL) is dissolved in an organic solvent, an amino acid and an alkaline catalyst are added to an organic solvent, and then the resulting mixture is added to the GDL solution to react at 55-60 °C after the amino acid and the alkaline catalyst are completely dissolved, after the reaction is finished, a white precipitate is filtered out, and the filtrate is evaporated to dryness to give a crude product.

According to the preparation method disclosed but not claimed herein, the organic solvent may be selected from methanol, ethanol and the like.

In one embodiment of the disclosure (not claimed), the glycopeptide derivative is prepared by using a solid-phase synthesis method comprising: resin swelling, covalently linking an amino acid where the amino group is protected to the swollen resin, deprotecting, adding a saccharide (such as ring-opened GDL) for condensation reaction, cleavaging, and purifying. GDL-L-Val and GDL-L-Ser were synthesized with reference to the method for synthesizing GDL-L-Thr.

The present disclosure further provides a peptidic compound (not claimed) of formula (9): 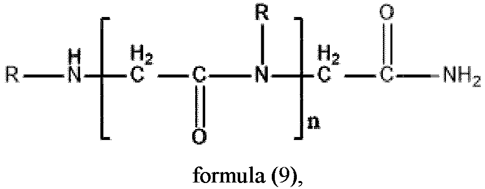 wherein R is selected from substituted or unsubstituted alkyl, and the substituent may be selected from -OH, -NH₂, -COOH, -CONH₂ and the like; for example, R is substituted or unsubstituted C₁₋₆ alkyl, and preferably R is -CH₃, -CH₂CH₃, -CH₂CH₂ COOH; n is an integer no less than 1 and no more than 1000, and for example, may be an integer ranging from 1 to 100. In some embodiments of the present invention, n is an integer such as 2, 3, 4, 5, 6, 7, 8, 9 and 10.

As an embodiment of the disclosure (not claimed), the compound of formula (9) has a structure shown in any one of the following:

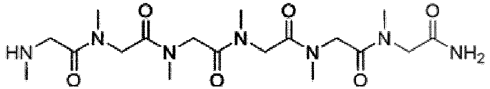

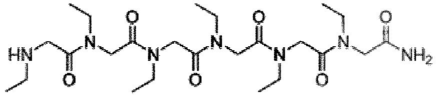

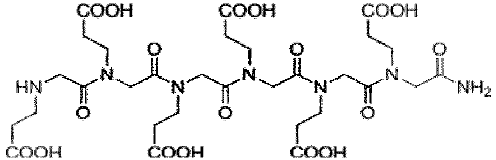

According to the present disclosure (not claimed), the compound of formula (9) is prepared by using the following synthetic route:

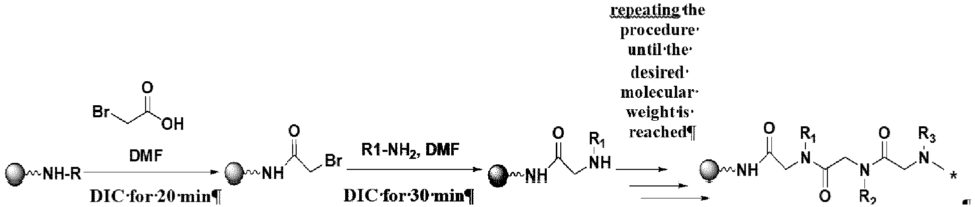

In an embodiment of the present disclosure (not claimed), the molecule of the ice growth inhibition material is an amino acid or a polyamino acid. The present disclosure further provides use (not claimed) of the above-mentioned molecule of the ice growth inhibition material, such as the PVA, the peptidic compound, the amino acid and the polyamino acid for controlling the growth of ice crystals in an aqueous solution, and use of the peptidic compound for preparing a cryopreservation solution for cells or tissues.

The ice growth inhibition materials designed according to the present invention, such as the PVA, the peptidic compound, the amino acid and the polyamino acid, can be used for preparing a cryopreservation solution for cryopreservation of cells, tissues, organs and the like, but such use or preparation does not form part of the claimed invention.

### [Example 1]

(1) Molecular structure design of compound:
   Compound molecules having the repeating unit -[CH₂-CHOH]- were designed to give a library for molecular structures that includes molecular models of an atactic and isotactic PVA.
(2) MD simulation experiment
   The differences in the affinities of the atactic PVA and the isotactic PVA for ice and water were predicted by MD simulation experiments.
   a. MD simulation was performed by GROMACS 5.1, and the model of water was TIP4P/2005, which has a melting point of about 252.5 K. The interaction parameters of the PVA molecules were provided by the GROMOS54A7 force field, and the leapfrog integration algorithm with an integration step size of 2 fs was adopted. The electrostatic interaction was calculated by the PME method, and the cutoff radii of the coulomb potential and the L-J potential were both 1.0 nm. A V-rescale (modified Berendsen) temperature-regulator and a pressure-regulator were used to regulate the temperature and pressure. The time constant was set to 0.1 ps.
   b. Molecular chains of compounds having 7 repeating units were simulated and selected for investigation. The topology files of the PVA molecules were generated through ATB, and in order to maintain the tacticities of the two PVA molecules, the dihedral angle potential functions of the carbon chains of the molecules were required to be adjusted correspondingly.
   c. Periodic boundary conditions were adopted for x-direction, y-direction and z-direction when an aqueous solution system of the PVAs was simulated; periodic boundary conditions were adopted for x-direction and y-direction when an ice-water mixed system was simulated. All systems were simulated for 120 ns, and data from the last 60 ns were used for analysis.

The aqueous solution system of the molecules was first investigated. In a system having only one PVA chain, 1491 water molecules in total were used, the pressure was 1 atm, and the temperatures were 240 K, 250 K, 260 K, 270 K, 300 K, and 330 K.

In a system for investigating interactions of PVA molecules with ice, 6 PVA molecular chains were placed in a box of water with dimensions of 3.9 × 3.6 × 1.0 nm³, an ice block comprising 1100 water molecules was equilibrated at 240 K for 10 ns, and the ice block was placed under the box of water along the z-axis. The size of the mixed system in the z-direction was increased to 10 nm, and the ice-water mixed system was placed at the center of the box of water.

The topology files of the PVA molecules were generated through ATB, and the topology files were directly used. In order to maintain the tacticities of the two PVA molecules, the potential energy parameters were set to be 50 kcal/mol, so that the molecular configurations of the two PVA molecules can be maintained even at a higher temperature.

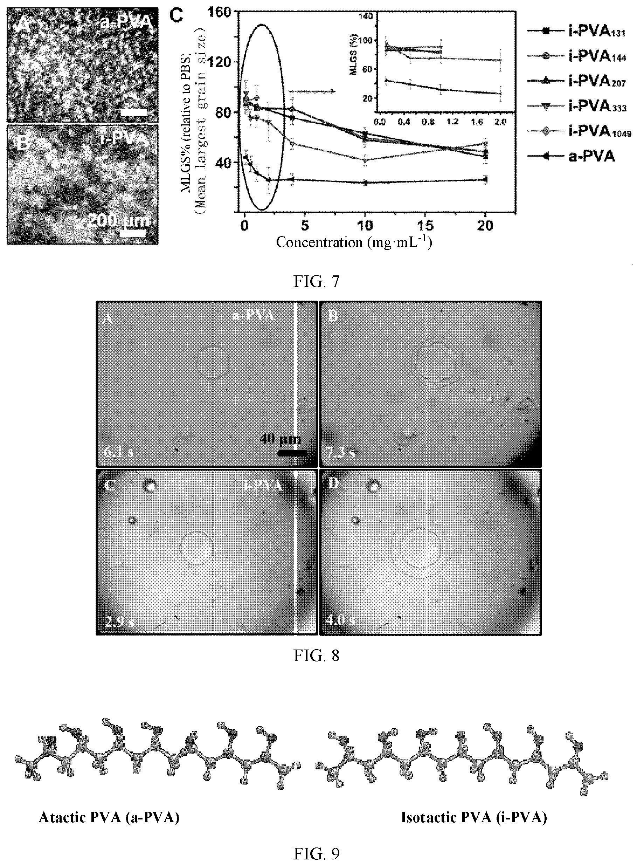

Molecular structure models of the two PVAs by MD simulation are shown in FIG. 9.

### (3) Evaluation of simulation

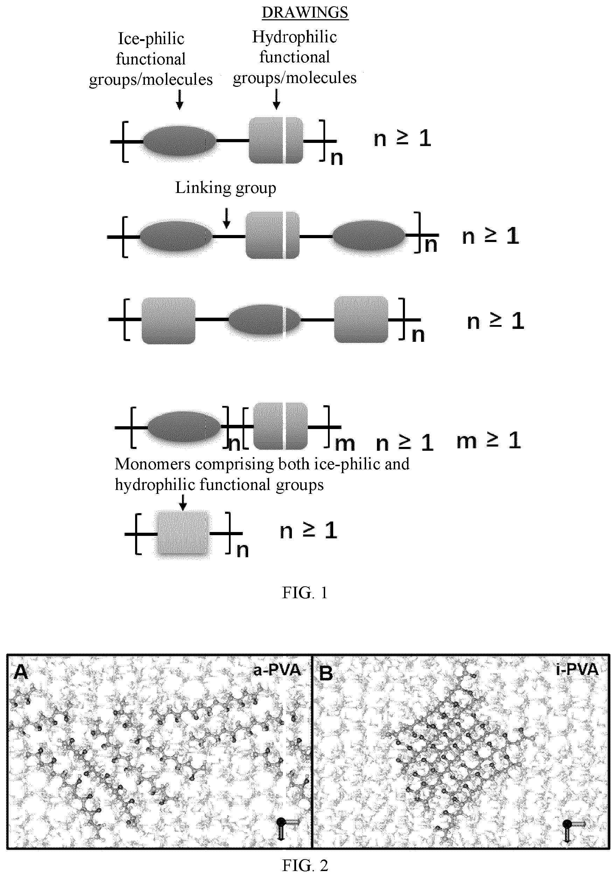

The a-PVA can effectively generate hydrogen bonding with an ice surface and thus be adsorbed on the ice surface because the distance of three times the distance between adjacent OH matches the size of the ice crystal lattice. The i-PVA only changes the direction of the hydroxyl group without changing the distance between adjacent OH, so that the i-PVA and the a-PVA have similar ice adsorption ability. Meanwhile, according to the MD simulation results, the number of the intermolecular hydrogen bonds formed between the a-PVA and water molecules is more than that of the intermolecular hydrogen bonds formed between the i-PVA and water molecules, which indicates that the affinity of the a-PVA for water is stronger than that of the i-PVA. In addition, the states of 6 PVA molecular chains at an ice-water interface simulated by the MD simulation show that, the a-PVA tends to spread at an ice-water interface due to its good affinities for both ice and water while the i-PVA tends to aggregate at an ice-water interface due to its weaker affinity for water (FIG. 2).

**Table 1**

| | a-PVA | | i-PVA | |
|---|---|---|---|---|
| T/K | Intramolecular hydrogen bonding | Intermolecular hydrogen bonding | Intramolecular hydrogen bonding | Intermolecular hydrogen bonding |
| 240 | 0.72(0.12) | 6.76(0.43) | 1.67(0.20) | 5.92(0.42) |
| 250 | 0.73(0.13) | 6.87(0.32) | 1.63(0.20) | 5.95(0.36) |
| 260 | 0.74(0.14) | 6.83(0.35) | 1.59(0.16) | 6.04(0.35) |
| 270 | 0.70(0.11) | 6.87(0.30) | 1.54(0.18) | 6.13(0.34) |
| 300 | 0.70(0.12) | 6.74(0.35) | 1.50(0.19) | 6.09(0.28) |
| 330 | 0.70(0.14) | 6.54(0.33) | 1.45(0.18) | 6.05(0.31) |

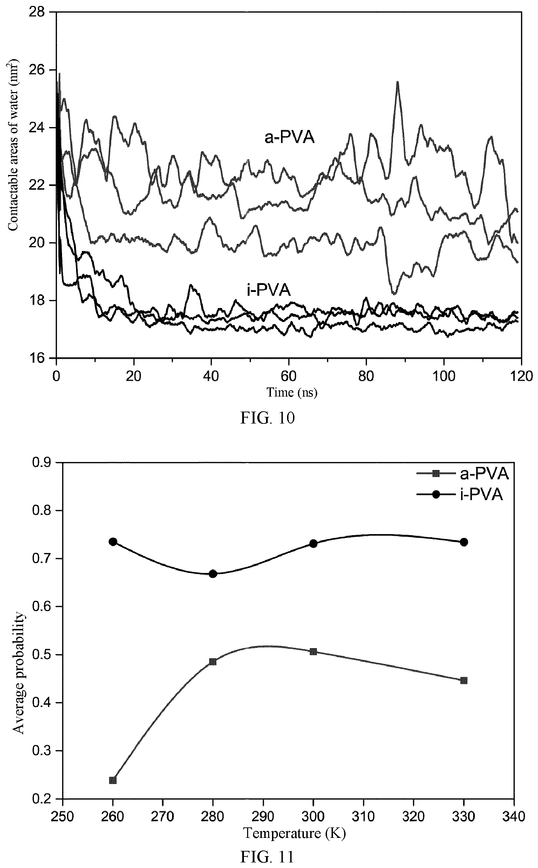

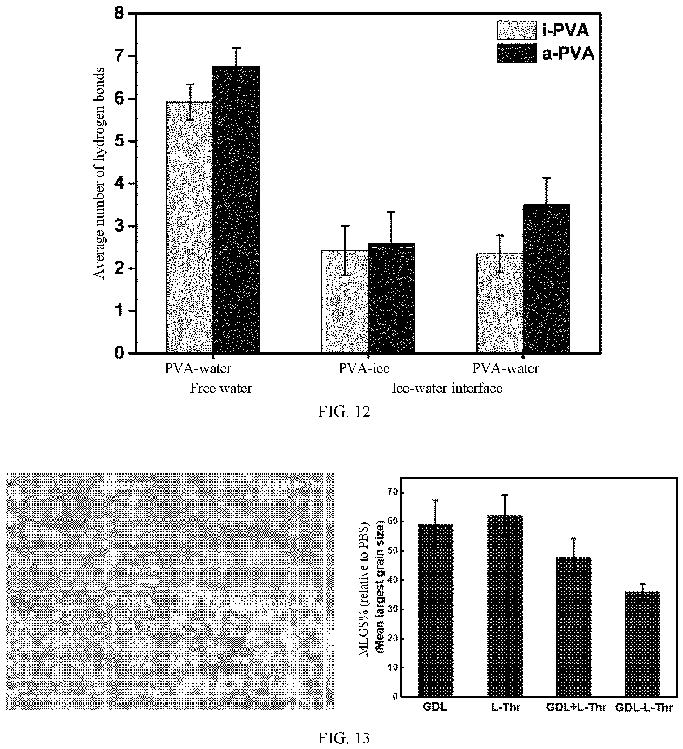

The MD simulation shows the contactable areas of the two PVAs with water molecules at an ice-water interface at 240 K, in which the contactable area of a-PVA is larger than that of the i-PVA, which further confirms that the spreading performance of the a-PVA at an ice-water interface is better than that of the i-PVA (see FIG. 10). The aggregation probability of the i-PVA in the aqueous solution calculated by MD is obviously higher than that of the a-PVA (FIG. 11); at 240K, the numbers of the hydrogen bonds formed between the two PVAs and ice-water molecules at an ice-water interface is similar, but the number of the hydrogen bonds formed between the a-PVA and water at an ice-water interface and in the aqueous solution is obviously more than that of the i-PVA. Therefore, the a-PVA can better spread at an ice-water interface while the i-PVA aggregates (FIG. 12).

Therefore, multiple results of the MD simulation show that the a-PVA has better spreading performance at an ice-water interface due to a strong affinity of its molecular structure for water molecules, and thus has a better ice growth inhibition effect than the i-PVA.

### (4) Synthesis of designed PVA molecules

### (4.1) Preparation of atactic polyvinyl alcohol a-PVA: the molecular weight is about 13-23 kDa, and the diad syndiotacticity r is about 55%

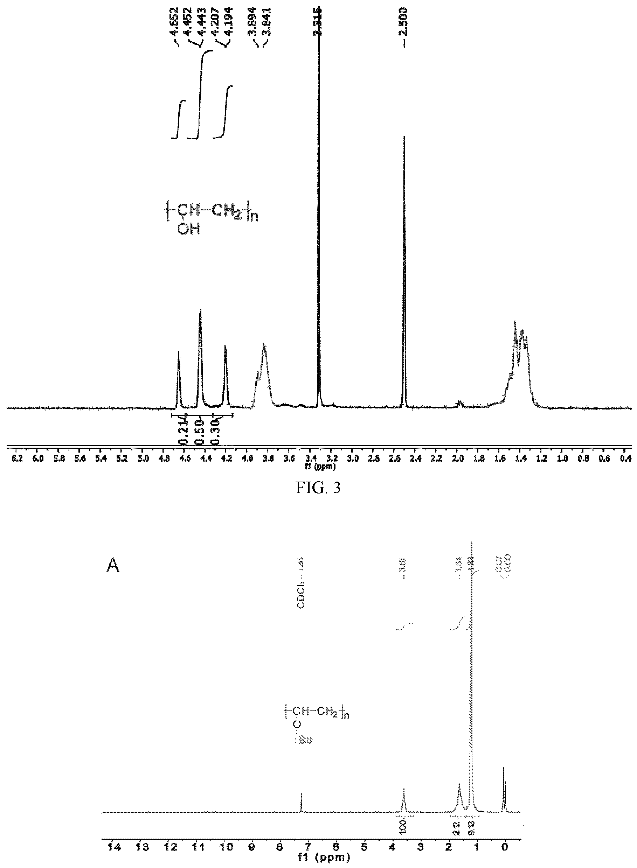

Vinyl acetate (VAc, Sigma-Aldrich) from which the inhibitor had been removed was dissolved in 100 mL of a solvent (methanol) in a 250 mL round-bottom flask under argon atmosphere to give a 25%-45% solution of VAc. After being cooled to -5 °C, the reaction solution was carefully added dropwise with 80 mM of 2,2'-Azobis(2-methylpropionitrile) (Sigma-Aldrich). After being left to warm to room temperature, the above solution was stirred for 15 h, and the reaction solution was dissolved with 1 L of acetone and added dropwise to methanol to give a white precipitate. The above precipitate was washed with methanol, filtered and dried in an oven at 60 °C under vacuum for 6.0 h to give a white solid. The white solid was dissolved in a methanol solution (10 wt.%), and argon gas was introduced to remove oxygen from the solution. A 25% methanol solution of potassium hydroxide was added dropwise to the above solution and stirred for 4 h. After the stirring, the reaction solution was dissolved in a 2 M hydrochloric acid solution and added to a 2.0 M methanol solution of ammonia for precipitatation to give an atactic polyvinyl alcohol (a-PVA). The proton NMR spectrum (FIG. 3) shows that the compound obtained is a fully hydrolyzed a-PVA.

### (4.2) Preparation of isotactic polyvinyl alcohol i-PVA: the molecular weight is about 14-26 kDa, and the isotacticity m is about 84%

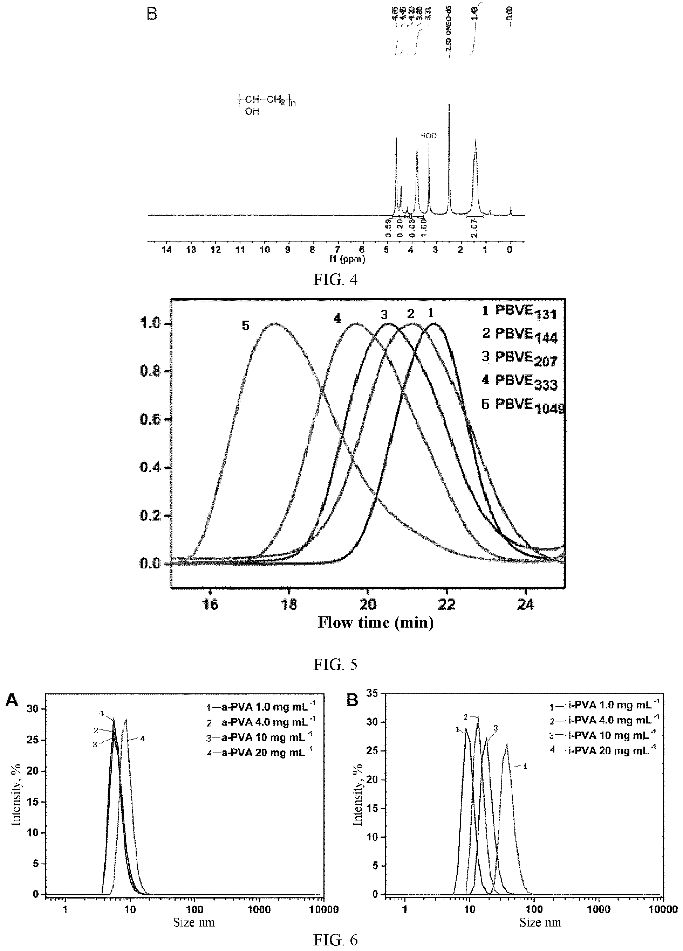

a. Preparation of poly-*tert*-butyl vinyl ether (PBVE). *Tert-*butyl vinyl ether (t-BVE, Sigma-Aldrich) was dissolved in 100 mL of dry toluene in a 250 mL round-bottom flask under argon atmosphere to give a 2.5% toluene solution of *t*-BVE. After being cooled to -78 °C, the above solution was carefully added dropwise with 0.2 mM boron trifluoride diethyl ether (BF₃·OEt₂, Sigma-Aldrich), and supplemented with 0.2 mM BF₃·OEt₂ 2.0 h later. After the above solution was stirred at -78 °C for 3.0 h, the reaction was stopped with a small amount of methanol. The reaction solution was added dropwise to 2.0 L of methanol with rapid stirring to give a light yellow precipitate. The precipitate was washed with methanol, filtered and dried in an oven at 60 °C under vacuum for 6.0 h to give a light yellow solid powder, which was PBVE as shown in the proton NMR spectrum (FIG. 4A). The molecular weight of the synthesized PBVE was regulated by adjusting the concentrations of boron trifluoride diethyl ether and *tert-*butyl vinyl ether. PBVE with different molecular weights was successfully synthesized as shown by the gel permeation chromatography (GPC) chromatogram (obtained using tetrahydrofuran (THF) system, flow rate of 1 mL·min⁻¹) (FIG. 5).
b. Preparation of dry hydrogen bromide gas (HBr); in a 100 mL two-neck flask, 5.0-30 mL of phosphorus tribromide (PBr₃, Aladdin) was added dropwise to 10 mL of 48% aqueous solution of hydrogen bromide (HBr, Alfa Aesar). The resulting gas was allowed to sequentially pass through tetrachloromethane (CCl₄), red phosphorus (P, Alfa Aesar) and calcium chloride (CaCl₂) to give a dry HBr gas.
c. Preparation of isotactic polyvinyl alcohol (isotactic-PVA, i-PVA). 0.5 g of PBVE was dissolved in 15 mL of dry toluene under argon atmosphere, and dry argon was continuously introduced to remove oxygen from the resulting solution. The dry HBr gas prepared in the step b was allowed to pass into the above oxygen-free toluene solution of PBVE at 0 °C. After about 5.0 min, a light yellow precipitate formed, and the introduction of dry HBr gas was continued until no precipitate formed. The above reaction solution was poured into 200 mL of methanol solution of ammonia (2.0 M).The resulting precipitate was washed with methanol, filtered, and dried in an oven at 60 °C under vacuum for 6.0 h to give a light yellow solid powder. The proton NMR spectrum (FIG. 4B) shows that the hydrolysis of PBVE was complete to give a solid i-PVA.

### (5) Verification of ice growth inhibition effect of synthesized PVA

### (5.1) Dynamic light scattering (DLS) experiment

The grain size distributions of the two PVAs (a-PVA: the molecular weight of about 13-23 kDa, the diad syndiotacticity r of about 55% (Sigma-Aldrich); i-PVA: the molecular weight of about 14-26 kDa, the isotacticity m of about 84%) in an aqueous solution at 25 °C were measured by a DLS experiment, and an experimental instrument was a Nano ZS (Malvern Instruments) with a thermostatic chamber and a 4 mW He-Ne laser (λ = 632.8 nm), wherein the scattering angle is 173°. Firstly, aqueous solutions of the a-PVA and the i-PVA at the concentrations of 1.0 mg·mL⁻¹, 4.0 mg·mL⁻¹, 10 mg·mL⁻¹ and 20 mg·mL⁻¹ were prepared; about 1.0 mL of each the PVA solution was added into a 12 mm disposable polystyrene cuvette for measurement.

The results of the DLS experiment show that when being at the same concentration, the a-PVA has a much smaller dispersion size in an aqueous solution than the i-PVA (FIG. 6). That is, compared to the a-PVA, the i-PVA tends to exist in an aggregated state in an aqueous solution. This is consistent with the results that in the MD simulation, the number of the intramolecular hydrogen bonds of the a-PVA is less than that of the i-PVA, and the number of the intermolecular hydrogen bonds formed between the a-PVA and water molecules is more than that formed between the i-PVA and water molecules.

### (5.2) Assay for ice recrystallization inhibition (IRI) activity

The IRI activity was assessed using "splat-freezing method", wherein a sample was dissolved and dispersed into a DPBS solution, and 10-30 µL of the resulting solution was added dropwise onto the surface of a clean silicon disk pre-cooled at -60 °C at a height of no less than 1.0 m; the solution was slowly heated to -6 °C at a speed of 10 °C·min⁻¹ by using a hot-cold stage, and was annealed for 30 min at this temperature; the sizes of ice crystals were observed and recorded by using a polarizing microscope and a high-speed camera. The hot-cold stage was sealed to ensure that the internal humidity was about 50%. The procedure was repeated at least three times for each sample, and the sizes of ice crystals were counted using a Nano Measurer 1.2, with the error of the result being the standard deviation.

### (5.3) Ice topography (DIS) observation and thermal hysteresis (TH) measurement

DIS observation and TH measurement were performed by using a nanoliter osmometer. A capillary was first melt with the outer flame of a alcohol burner, and simultaneously stretched to produce a capillary with a very fine pore size, and the capillary was then linked to a microsyringe; an immersion oil with higher viscosity was injected into a disk with micron-sized holes, and an aqueous solution in which the material was dissolved was injected into the microholes by using a microsyringe; the droplet was quickly frozen by regulating the temperature, and then slowly heated to give a single crystal ice, the single crystal ice was slowly cooled at the precision of 0.01 °C, and the DIS observation and TH test were performed by using a microscope provided with a high-speed camera.

The ability of the a-PVA (*M*_{w} 13-23 kD) to inhibit the growth of ice crystals is far better than that of the i-PVA (*M*_{w} 14-26 kD) with the corresponding molecular weight (FIG. 7). As can be seen in FIG. 7A, the grain size of the a-PVA is obviously smaller than that of the i-PVA at the same concentration; as can be seen in FIG. 7B, the mean largest grain size (MLGS) of the ice crystal of the a-PVA relative to that of DPBS reaches a minimum after 2.0 mg·mL⁻¹, the minimum being about 20% of the MLGS of the ice crystal of DPBS; the MLGS of the i-PVA of different molecular weights relative to that of DPBS increases with the increasing concentration and reaches a minimum at 10 mg mL⁻¹, the minimum being only about 50% of the MLGS of the ice crystal of DPBS, and the MLGS does not decrease but increase slightly with the concentration continuing to increase to 20 mg mL⁻¹. The i-PVA (*M*_{w} 14-26 kD) with the degree of polymerization of more than 333 was difficult to dissolve at the concentration of more than 30 mg mL⁻¹. Therefore, due to the limitation of the solubility of the i-PVA, the IRI activity of the i-PVA was optimally 50% of the MLGS of DPBS when the concentration was 10 mg mL⁻¹, and the IRI activity of the a-PVA is optimally 20% of the MLGS of DPBS when the concentration was 2.0 mg mL⁻¹. This is consistent with the results that the a-PVA spreads more easily at an ice-water interface than the i-PVA in the MD simulation, which achieves the effect that the a-PVA can better inhibit the growth of ice crystals at a lower dosage than the i-PVA.

As can be seen from the results of the MD simulation and the actual verification experiment, the results are good in consistency. The ice growth inhibition performance of the ice growth inhibition material can be accurately predicted by MD simulation, and the molecular design of the ice growth inhibition material can be effectively achieved.

Compounds of formula (1) to formula (9) were designed by the same molecular design method, synthesized and studied for their ice growth inhibition effects.

### [Example 2] Synthesis of compound of formula (1)

(1) 2-chlorotrityl chloride resin was placed into a reaction tube, and added with DCM (20 mL·g⁻¹). The resulting mixture was shaken for 30 min. With the use of a sand-core funnel by suction, the solvent was removed. The residue was added with a three-fold molar excess of Fmoc-L-Pro-OH and an eight-fold molar excess of DIEA, and finally added with DMF to dissolve. The resulting mixture was shaken for 30 min. Methanol was used for end-capping for 30 min.
(2) The solvent DMF was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol three times, added with a ninhydrin reagent, and heated at 105-110 °C for 5 min. The color turned dark blue, which suggested a positive reaction.
(3) After the product obtained by the above reaction was sequentially washed with DMF (15 mL·g⁻¹, twice), methanol (15 mL·g⁻¹, twice) and DMF (15 mL·g⁻¹, twice), a two-fold excess of Fmoc-L-Thr(tBu)-OH that was dissolved in as small an amount of DMF as possible was added to a reaction tube; a two-fold excess of HBTU was added. Immediately thereafter, an eight-fold excess of DIEA was added and reacted for 30 min.
(4) After the solution was removed by suction, a small amount of resin was taken and washed with ethanol three times, added with a ninhydrin reagent, and heated at 105-110 °C for 5 min. The colorless mixture suggested a negative reaction, that is, the reaction was complete.
(5) After the product obtained by the above reaction was sequentially washed with DMF (15 mL·g⁻¹, twice), methanol (15 mL·g⁻¹, twice) and DMF (15 mL·g⁻¹, twice), the solvent was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol, added with a ninhydrin reagent, and heated at 105-110 °C for 5 min. The color turned dark blue, which suggested a positive reaction.
(6) After the product obtained by the above reaction was sequentially washed with DMF (15 mL·g⁻¹, twice), methanol (15 mL·g⁻¹, twice) and DCM (15 mL·g⁻¹, twice), the resin was dried by suction.
(7) The product was cleavaged using a cleavaging liquid (15 mL·g⁻¹, TFA:water:EDT:Tis = 95:1:2:2, v/v) for 90 min. The cleavaging fluid was blown to dryness with nitrogen, and then frozen to dryness to give a crude product of polypeptide.
(8) The polypeptide was purified and subjected to salt-conversion or desalting by HPLC. HPLC: tR = 6.1 mins (purification column model: Kromasil 100-5C18, 4.6 mm*250 mm; gradient eluent: acetonitrile with 0.1% TFA and aqueous solution with 0.1% TFA, 0 mins-1:99, 20 mins-1:9). The purified solution was frozen to dryness to give a purified product L-Thr-L-Pro (indicated as TP). The yield was about 80%. The mass spectrum presents [M+H]⁺ at 217.3.

### [Example 3] Synthesis of compound of formula (2)

(1) 2-chlorotrityl chloride resin was placed into a reaction tube, and added with DCM (20 mL·g⁻¹). The resulting mixture was shaken for 30 min. With the use of a sand-core funnel by suction, the solvent was removed. The residue was added with a three-fold molar excess of Fmoc-L-Thr(tBu)-OH and an eight-fold molar excess of DIEA, and finally added with DMF to dissolve. The resulting mixture was shaken for 30 min. Methanol was used for end-capping for 30 min.
(2) The solvent DMF was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol three times, added with a ninhydrin reagent, and heated at 105-110 °C for 5 min. The color turned dark blue, which suggested a positive reaction.
(3) After the product obtained by the above reaction was sequentially washed with DMF (15 mL·g⁻¹, twice), methanol (15 mL·g⁻¹, twice) and DMF (15 mL·g⁻¹, twice), a two-fold excess of Fmoc-Arg(Pbf)-OH that was dissolved in as small an amount of DMF as possible was added to a reaction tube; a two-fold excess of HBTU was added. Immediately thereafter, an eight-fold excess of DIEA was added and reacted for 30 min.
(4) After the solution was removed by suction, a small amount of resin was taken and washed with ethanol three times, added with a ninhydrin reagent, and heated at 105-110 °C for 5 min. The colorless mixture suggested a negative reaction, that is, the reaction was complete.
(5) After the product obtained by the above reaction was sequentially washed with DMF (15 mL·g⁻¹, twice), methanol (15 mL·g⁻¹, twice) and DMF (15 mL·g⁻¹, twice), the solvent was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol, added with a ninhydrin reagent, and heated at 105-110 °C for 5 min. The color turned dark blue, which suggested a positive reaction.
(6) After the product obtained by the above reaction was sequentially washed with DMF (15 mL·g⁻¹, twice), methanol (15 mL·g⁻¹, twice) and DCM (15 mL·g⁻¹, twice), the resin was dried by suction.
(7) The product was cleavaged using a cleavaging liquid (15 mL·g⁻¹, TFA:water:EDT:Tis = 95:1:2:2, v/v) for 90 min. The cleavaging fluid was blown to dryness with nitrogen, and then frozen to dryness to give a crude product of polypeptide.
(8) The polypeptide was purified and subjected to salt-conversion or desalting by HPLC. HPLC: tR = 4.8 mins (purification column model: Kromasil 100-5C18, 4.6 mm*250 mm; gradient eluent: acetonitrile with 0.1% TFA and aqueous solution with 0.1% TFA, 0 mins-1:99, 20 mins-1:4). The purified solution was frozen to dryness to give a purified product L-Thr-L-Arg (TR). The yield was about 80%. The mass spectrum presents [M+H]⁺ at 276.2.

### [Example 4] Synthesis of compound of formula (3)

(1) 2-chlorotrityl chloride resin was placed into a reaction tube, and added with DCM (20 mL·g⁻¹). The resulting mixture was shaken for 30 min. With the use of a sand-core funnel by suction, the solvent was removed. The residue was added with a three-fold molar excess of Fmoc-L-Thr(tBu)-OH and an eight-fold molar excess of DIEA, and finally added with DMF to dissolve. The resulting mixture was shaken for 30 min. Methanol was used for end-capping for 30 min.
(2) The solvent DMF was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol three times, added with a ninhydrin reagent, and heated at 105-110 °C for 5 min. The color turned dark blue, which suggested a positive reaction.
(3) After the product obtained by the above reaction was sequentially washed with DMF (15 mL·g⁻¹, twice), methanol (15 mL·g⁻¹, twice) and DMF (15 mL·g⁻¹, twice), a two-fold excess of Fmoc-Arg(Pbf)-OH that was dissolved in as small an amount of DMF as possible was added to a reaction tube; a two-fold excess of HBTU was added. Immediately thereafter, an eight-fold excess of DIEA was added and reacted for 30 min.
(4) After the solution was removed by suction, a small amount of resin was taken and washed with ethanol three times, added with a ninhydrin reagent, and heated at 105-110 °C for 5 min. The colorless mixture suggested a negative reaction, that is, the reaction was complete.
(5) After the product obtained by the above reaction was sequentially washed with DMF (15 mL·g⁻¹, twice), methanol (15 mL·g⁻¹, twice) and DMF (15 mL·g⁻¹, twice), the solvent was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol, added with a ninhydrin reagent, and heated at 105-110 °C for 5 min. The color turned dark blue, which suggested a positive reaction.
(6) After the product obtained by the above reaction was sequentially washed with DMF (15 mL·g⁻¹, twice), methanol (15 mL·g⁻¹, twice) and DMF (15 mL·g⁻¹, twice), the resin was dried by suction.
(7) Steps (3) to (5) were repeated to link amino acid Fmoc-L-Thr(tBu)-OH. After the product obtained by the reaction was sequentially washed with DMF (15 mL·g⁻¹, twice), methanol (15 mL·g⁻¹, twice) and DCM (15 mL·g⁻¹, twice), the resin was dried by suction.
(8) The product was cleavaged using a cleavaging liquid (15 mL·g⁻¹, TFA:water:EDT:Tis = 95:1:2:2, v/v) for 90 min. The cleavaging fluid was blown to dryness with nitrogen, and then frozen to dryness to give a crude product of polypeptide.
(9) The polypeptide was purified and subjected to salt-conversion or desalting by HPLC. HPLC: tR = 3.9 mins (purification column model: Kromasil 100-5C18, 4.6 mm*250 mm; gradient eluent: acetonitrile with 0.1% TFA and aqueous solution with 0.1% TFA, 0 mins-1:99, 20 mins-1:4). The purified solution was frozen to dryness to give a purified product L-Thr-L-Arg-L-Thr (TRT). The yield was about 75%. The mass spectrum presents [M+H]⁺ at 377.4.

### [Example 5] Synthesis of compound of formula (4)

(1) 2-chlorotrityl chloride resin was placed into a reaction tube, and added with DCM (20 mL·g⁻¹). The resulting mixture was shaken for 30 min. With the use of a sand-core funnel by suction, the solvent was removed. The residue was added with a three-fold molar excess of Fmoc-L-Thr(tBu)-OH and an eight-fold molar excess of DIEA, and finally added with DMF to dissolve. The resulting mixture was shaken for 30 min. Methanol was used for end-capping for 30 min.
(2) The solvent DMF was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol three times, added with a ninhydrin reagent, and heated at 105-110 °C for 5 min. The color turned dark blue, which suggested a positive reaction.
(3) After the product obtained by the above reaction was sequentially washed with DMF (15 mL·g⁻¹, twice), methanol (15 mL·g⁻¹, twice) and DMF (15 mL·g⁻¹, twice), a two-fold excess of Fmoc-L-Pro-OH that was dissolved in as small an amount of DMF as possible was added to a reaction tube; and a two-fold excess of HBTU was added. Immediately thereafter, an eight-fold excess of DIEA was added and reacted for 30 min.
(4) After the solution was removed by suction, a small amount of resin was taken and washed with ethanol three times, added with a ninhydrin reagent, and heated at 105-110 °C for 5 min. The colorless mixture suggested a negative reaction, that is, the reaction was complete.
(5) After the product obtained by the above reaction was sequentially washed with DMF (15 mL·g⁻¹, twice), methanol (15 mL·g⁻¹, twice) and DMF (15 mL·g⁻¹, twice), the solvent was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol, added with a ninhydrin reagent, and heated at 105-110 °C for 5 min. The color turned dark blue, which suggested a positive reaction.
(6) After the product obtained by the above reaction was sequentially washed with DMF (15 mL·g⁻¹, twice), methanol (15 mL·g⁻¹, twice) and DMF (15 mL·g⁻¹, twice), the resin was dried by suction.
(7) Steps (3) to (5) were repeated to link amino acid Fmoc-L-Thr(tBu)-OH. After the product obtained by the reaction was sequentially washed with DMF (15 mL·g⁻¹, twice), methanol (15 mL·g⁻¹, twice) and DCM (15 mL·g⁻¹, twice), the resin was dried by suction.
(8) The product was cleavaged using a cleavaging liquid (15 mL·g⁻¹, TFA:water:EDT:Tis = 95:1:2:2, v/v) for 90 min. The cleavaging fluid was blown to dryness with nitrogen, and then frozen to dryness to give a crude product of polypeptide.
(9) The polypeptide was purified and subjected to salt-conversion or desalting by HPLC. HPLC: tR = 7.6 mins (purification column model: Kromasil 100-5C18, 4.6 mm*250 mm; gradient eluent: acetonitrile with 0.1% TFA and aqueous solution with 0.1% TFA, 0 mins-1:99, 20 mins-2:8). The purified solution was frozen to dryness to give a purified product L-Thr-L-Pro-L-Thr (TPT). The yield was about 70%. The mass spectrum presents [M+H]⁺ at 318.3.

### [Example 6] Synthesis of compound of formula (5)

(1) 2-chlorotrityl chloride resin was placed into a reaction tube, and added with DCM (20 mL·g⁻¹). The resulting mixture was shaken for 30 min. With the use of a sand-core funnel by suction, the solvent was removed. The residue was added with a three-fold molar excess of Fmoc-L-Thr(tBu)-OH and an eight-fold molar excess of DIEA, and finally added with DMF to dissolve. The resulting mixture was shaken for 30 min. Methanol was used for end-capping for 30 min.
(2) The solvent DMF was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol three times, added with a ninhydrin reagent, and heated at 105-110 °C for 5 min. The color turned dark blue, which suggested a positive reaction.
(3) After the product obtained by the above reaction was sequentially washed with DMF (15 mL·g⁻¹, twice), methanol (15 mL·g⁻¹, twice) and DMF (15 mL·g⁻¹, twice), a two-fold excess of Fmoc-L-Ala-OH that was dissolved in as small an amount of DMF as possible was added to a reaction tube; a two-fold excess of HBTU was added. Immediately thereafter, an eight-fold excess of DIEA was added and reacted for 30 min.
(4) After the solution was removed by suction, a small amount of resin was taken and washed with ethanol three times, added with a ninhydrin reagent, and heated at 105-110 °C for 5 min. The colorless mixture suggested a negative reaction, that is, the reaction was complete.
(5) After the product obtained by the above reaction was sequentially washed with DMF (15 mL·g⁻¹, twice), methanol (15 mL·g⁻¹, twice) and DMF (15 mL·g⁻¹, twice), the solvent was removed. 20% piperidine/DMF solution (10 mL·g⁻¹) was added, and the solvent was removed after 5 min; 20% piperidine/DMF solution (10 mL·g⁻¹) was added again, and the piperidine solution was removed after 15 min. A small amount of resin was taken and washed with ethanol, added with a ninhydrin reagent, and heated at 105-110 °C for 5 min. The color turned dark blue, which suggested a positive reaction.
(6) After the product obtained by the above reaction was sequentially washed with DMF (15 mL·g⁻¹, twice), methanol (15 mL·g⁻¹, twice) and DMF (15 mL·g⁻¹, twice), the resin was dried by suction.
(7) Steps (3) to (5) were repeated to link amino acid Fmoc-L-Ala-OH. After the product obtained by the reaction was sequentially washed with DMF (15 mL·g⁻¹, twice), methanol (15 mL·g⁻¹, twice) and DCM (15 mL·g⁻¹, twice), the resin was dried by suction.
(8) The product was cleavaged using a cleavaging liquid (15 mL·g⁻¹, TFA:water:EDT:Tis = 95:1:2:2, v/v) for 90 min. The cleavaging fluid was blown to dryness with nitrogen, and then frozen to dryness to give a crude product of polypeptide.
(9) The polypeptide was purified and subjected to salt-conversion or desalting by HPLC. HPLC: tR = 7.9 mins (purification column model: Kromasil 100-5C18, 4.6 mm*250 mm; gradient eluent: acetonitrile with 0.1% TFA and aqueous solution with 0.1% TFA, 0 mins-1:99, 20 mins-1:9). The purified solution was frozen to dryness to give a purified product L-Ala-L-Ala-L-Thr (AAT). The yield was about 70%. The mass spectrum presents [M-8H]⁺ at 260.1.

### [Example 7] Synthesis of compounds of formula (6), formula (7) and formula (8)

### Preparation of a compound of formula (6):

(1) GDL-L-Thr was prepared by using a solid-phase synthesis method.
(2) Purification by HPLC. HPLC: tR = 3.4 mins (purification column type: SHIMADZU Intertsil ODS-SP (4.6 mm*250 mm*5 µM), gradient eluent: acetonitrile with 0.1% TFA and aqueous solution with 0.1% TFA, 0.01-20 mins-1:99, 20-30 mins-21:79, 30-40 mins-100:0, 40-50 mins-1:99); the yield was about 50%. The mass spectrum presents [M-H]⁺ at 296.099.

The GDL-L-Thr prepared by using the solid-phase synthesis method has higher purity, and is more easily to separate. The experimental results show that the GDL-L-Thr prepared by using the solid-phase synthesis method has higher purity and good capability of inhibiting the growth of ice crystals (FIG. 13).

Compounds of both formulas (7) and (8) can be obtained by using a solid-phase synthesis method.

### [Example 8] Synthesis of compound of formula (9)

(1) A DCM solution of dichlorodimethylisilane was poured into a synthesis tube for polypeptide, and after standing for 30 min the tube was air-dried for later use.
(2) 100 mg of resin was placed into the synthesis tube, 2 mL of DMF was added, and nitrogen was introduced. The resin was swollen for 10 min and filtered by suction under vacuum.
(3) 1mL of 4-methylpiperidine/DMF solution was added for deprotection, and removed after 5 min. 1 mL of 4-methylpiperidine/DMF solution was added again, and removed after 15 min. The mixture was bubbled and filtered by suction under vacuum.
(4) The mixture was washed with DMF 5 times, bubbled and filtered by suction under vacuum.
(5) The mixture was sequentially added with 0.5 mL of 2 M bromoacetic acid/DMF solution and N,N-diisopropylcarbodiimide/DMF solution, bubbled for 20 min, filtered by suction under vacuum, and washed with DMF 3 times.
(6) The mixture was added with 1 mL of 1 M primary amine/DMF solution, bubbled for 30 min, washed with DMF, and washed with dichloromethane (×3).
(7) Steps (5) and (6) were repeated until the desired molecular weight was reached.
(8) The mixture was added with 4 mL of a cracking liquid, homogeneously mixed, blown to dryness with nitrogen, finally frozen to dryness, and purified to give the purified product.

In a peptoid, R is -CH₃, -CH₂CH₃ and -CH₂CH₂COOH. The mass spectrum presents [M+H]⁺ with R being -CH₃ at 444.6, [M+H]⁺ with R being -CH₂CH₃ at 528.8, and [M+H]⁺ with R being -CH₂CH₂COOH at 792.1.

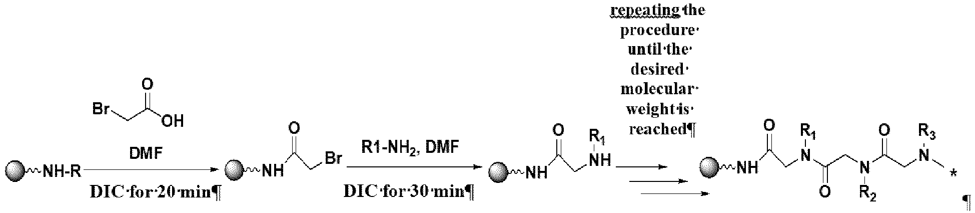

### [Ice recrystallization inhibition experiment]

The IRI activity was assessed using "splat-freezing method", wherein a sample was dissolved and dispersed into a DPBS solution, and 10-30 µL of the resultant solution was added dropwise onto the surface of a clean silicon disk precooled at -60 °C at a height of no less than 1.0 m; the solution was slowly heated to -6 °C at a speed of 10 °C/min by using a hot-cold stage, and was annealed for 30 min at this temperature; the sizes of ice crystals were observed and recorded by using a polarizing microscope and a high-speed camera. The hot-cold stage was sealed to ensure that the internal humidity was about 50%. The procedure was repeated at least three times for each sample, and the sizes of ice crystals were counted using a Nano Measurer 1.2, with the error of the result being the standard deviation.

DIS observation and TH measurement were performed by using a nanoliter osmometer. A capillary was first melt with the outer flame of a alcohol burner, and simultaneously stretched to produce a capillary with a very fine pore size, and the capillary was then linked to a microsyringe; an immersion oil with higher viscosity was injected into a disk with micron-sized holes, and an aqueous solution in which the material was dissolved was injected into the microholes by using a microsyringe; the droplet was quickly frozen by regulating the temperature, and then slowly heated to give a single crystal ice, the single crystal ice was slowly cooled at the precision of 0.01 °C, and the DIS observation and TH test were performed by using a microscope provided with a high-speed camera.

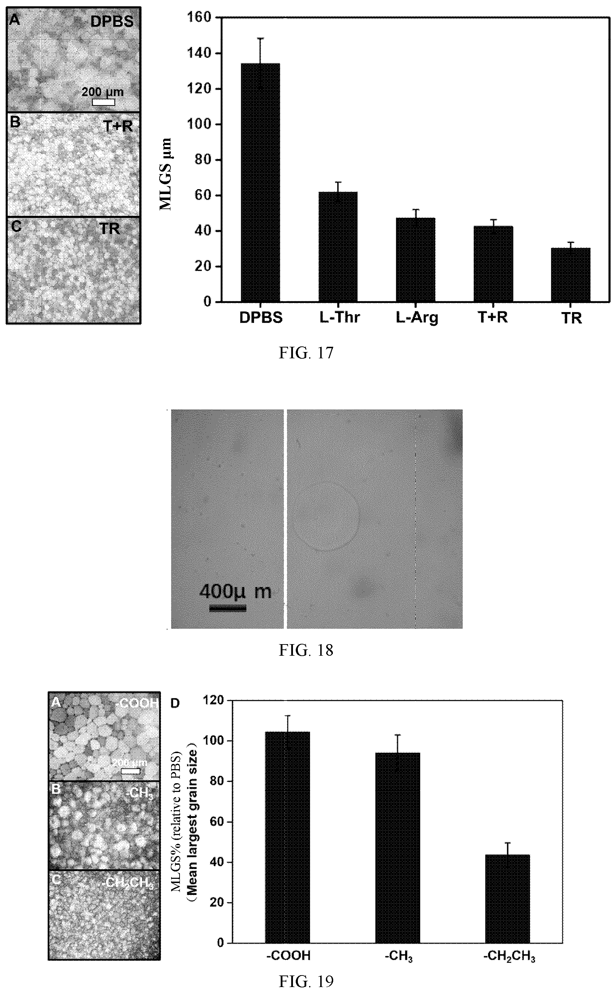

An IRI activity test was performed on 20 µL of a DPBS solution of the TR prepared in Example 3 by using "splat-freezing method". The determined MLGS (%) relative to that of DPBS is shown in FIG. 17. The MLGS of the TR bound by chemical bonds is obviously smaller than that of the DPBS solutions of arginine and threonine at the same concentration.

The deionized aqueous solution of the TR prepared in Example 3 was taken for DIS observation using a nanoliter osmometer. It was found that the TR had a weak modification effect on the topography of ice crystals (supercooling degree of -0.1 °C, -0.4 to 0.01 °C), as shown in FIG. 18. No TH was determined.

IRI activity tests were performed on 20 µL of DPBS solutions of the GDL-L-Thr, GDL-L-Ser and GDL-L-Val prepared in Example 7 by using "splat-freezing method". The determined MLGS (%) relative to that of DPBS is shown in FIG. 13 and FIGs. 15-16. The MLGSs of the GDL-L-Thr, GDL-L-Ser and GDL-L-Val bound by chemical bonds are obviously smaller than those of the DPBS solutions of GDL and amino acids at the same concentration, and small than those of the DPBS solutions of mixtures of GDL and amino acids at the same concentration.

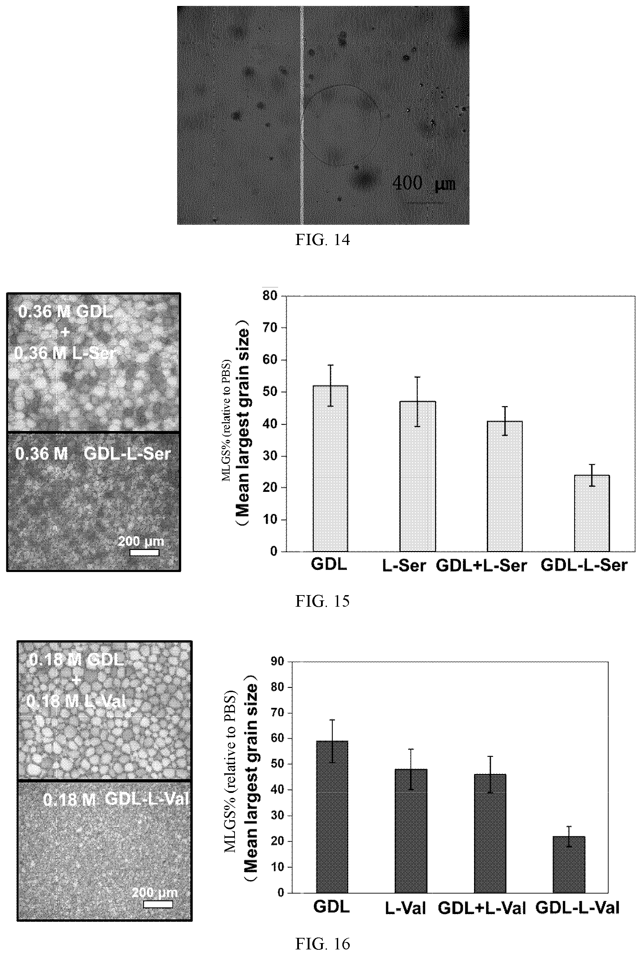

The deionized aqueous solution of the GDL-L-Thr prepared in Example 7 was taken for DIS observation using a nanoliter osmometer. It was found that the GDL-L-Thr had a weak modification effect on the topography of ice crystals (supercooling degree of -0.1 °C, -0.4 to 0.01 °C), as shown in FIG. 14. No TH was determined.

IRI activity tests were performed on 20 µL of DPBS solutions of the compounds prepared in Example 8 by using "splat-freezing method". The determined MLGS (%) relative to that of DPBS is shown in FIG. 19.

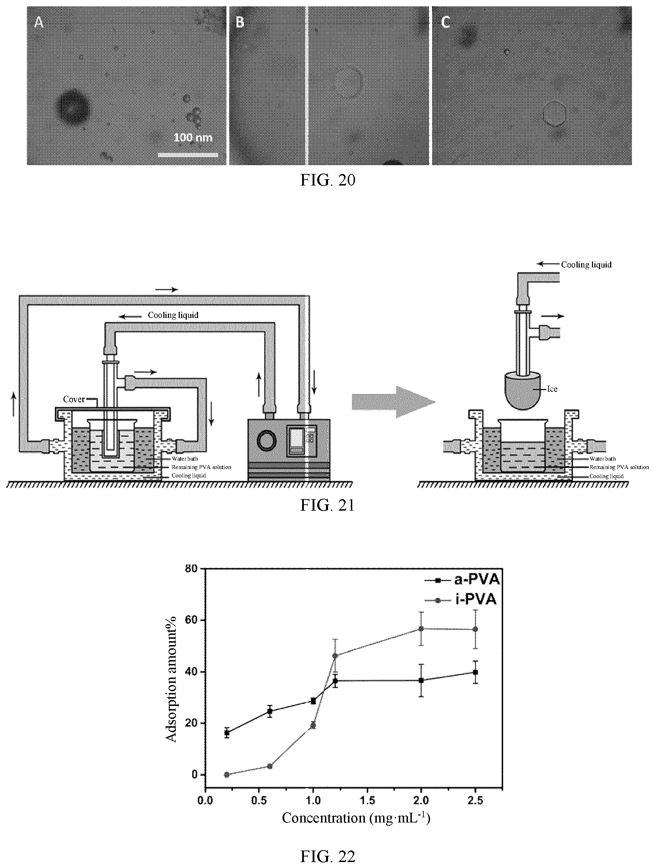

The deionized aqueous solutions of the three peptoids prepared in Example 8 were taken for DIS observation using a nanoliter osmometer. It was found that the peptoids where R was -CH₃ and -CH₂CH₃ had rather obvious modification effects on the topography of ice crystals, and the peptoids where R was -CH₂CH₂COOH had no modification effect on the topography of ice crystals (supercooling degree of -0.1 °C, -0.4 to 0.01 °C). The topography obtained are shown in FIG. 20, and no TH was detected for the three peptoids.

The above results show that the prepared peptidic compounds have the activity to inhibit the growth of ice crystals and have modification effects on the topography of ice crystals, particularly the compound of formula (9) where R is -CH₃ or -CH₂CH₃ has an excellent modification effect on the topography of ice crystals with no TH, and can achieve the effect of controlling the growth of ice crystals and be used in the cryopreservation solution.

### B. Ice growth inhibition performance evaluation and screening of ice growth inhibition material

The amount of the ice growth inhibition material adsorbed on an ice surface = (the mass of ice growth inhibition material adsorbed on ice surface m₁/total mass of ice growth inhibition material in stock solution comprising ice growth inhibition material m₂) × 100%.

In one embodiment, the ice adsorption experiment comprises the following steps:
S1, taking an ice growth inhibition material of the mass m₂ to prepare an aqueous solution of the ice control material, and cooling to a supercooling temperature;
S2, placing a pre-cooled temperature-regulating rod into the aqueous solution to induce the growth of an ice layer on the surface of the temperature-regulating rod, continuously stirring the aqueous solution to enable the ice growth inhibition material to be gradually adsorbed onto the surface of the ice layer, and keeping the temperature of the aqueous solution and the temperature-regulating rod at a supercooling temperature; and
S3, determining the amount of the ice growth inhibition material absorbed on the ice surface.

The device shown in FIG. 21 was used to perform the ice adsorption experiment. The device comprises a multilayer liquid storage cavity, a temperature-regulating rod and a temperature regulator, wherein the multilayer liquid storage cavity sequentially comprises an ice adsorption cavity, a bath cavity and a cooling liquid storage cavity from the inside to the outside, the temperature-regulating rod being arranged in the ice adsorption cavity, and the temperatures of the temperature-regulating rod and the liquid storage cavity being regulated by the temperature regulator. The temperature-regulating rod is of a hollow structure made of a thermally conductive material, and the hollow structure of the temperature-regulating rod is provided with a liquid inlet and a liquid outlet; the temperature regulator is a fluid temperature regulator and is provided with a cooling liquid outflow end and a reflux end; two ends of the cooling liquid storage cavity is provided with a liquid inlet and a liquid outlet; the cooling liquid outflow end of the temperature regulator, the liquid inlet of the temperature-regulating rod, the liquid outlet of the temperature-regulating rod, the liquid inlet of a cooling liquid storage tank, the liquid outlet of the cooling liquid storage tank and the reflux end of the temperature regulator are sequentially linked via pipelines through which the cooling liquid flows. The multilayer liquid storage cavity is provided with a cover. When in use, the ice adsorption cavity is arranged to contain an aqueous solution of the ice growth inhibition material, and the bath cavity in the middle layer is arranged to contain a bath medium that is at a preset temperature, for example, a water bath, an ice bath or an oil bath; after the preset temperature of the cooling liquid is reached, the cooling liquid flows out through the temperature regulator and flows into the hollow structure of the temperature-regulating rod to regulate the temperature of the temperature-regulating rod, then flows out from the liquid outlet of the temperature-regulating rod and flows into the cooling liquid storage cavity in the outer layer to maintain the temperature of the bath medium at the preset level, and then flows through the liquid outlet of the cooling liquid storage tank and the reflux end of the temperature regulator and enters the temperature regulator to circulate.

### [Example 9]

a-PVA: molecular weight of about 13-23 kDa, diad syndiotacticity r of about 55% (Sigma-Aldrich);
i-PVA: molecular weight of about 14-26 kDa, isotacticity m of about 84%.

### (1) Measurement of spreading performance of two PVAs at ice-water interface

The amount of the PVAs adsorbed on an ice surface was determined by performing an ice adsorption experiment, and an experimental device is shown in FIG 21.
a. The a-PVA and the i-PVA were fluorescently labeled with FITC Isomer I.
b. The FITC-labeled aqueous solutions (40 mL) of the PVAs with different concentrations were placed in beakers, which were then placed in a recirculating cooling bath, and the temperatures of the solutions and the temperature-regulating rod were cooled to -0.1 °C.
c. The temperature-regulating rod was inserted into liquid nitrogen for pre-cooling for 1.0 min before being inserted into the pre-cooled FITC-labeled aqueous solutions of the PVAs. Then, the temperature-regulating rod was rapidly inserted into the pre-cooled FITC-labeled aqueous solutions of the PVAs to induce an extremely thin ice layer on the surface of the temperature-regulating rod to further induce ice growth.
d. The aqueous FITC-labeled PVA solution was magnetically stirred continuously at a supercooling temperature of -0.1 °C for 1.0 h to allow the PVA to be gradually adsorbed onto the surface of the ice. The supercooling degree and the adsorption time were maintained unchanged during all adsorption experiments to ensure that the surface area of the resulting ice was almost maintained unchanged within an allowable error range.
e. The formed ice block was taken out from the solution, and the ice surface was rinsed with purified water to remove the solution attached to the surface. The ice block was melted.
f. The amount of the PVA absorbed on the ice surface was obtained by the mass ratio of the solute PVA in the ice block to the solute PVA in the original solution, the concentration of the PVA solution was determined by ultraviolet-visible spectrophotometry, and the volume was determined by a pipette and a measuring cylinder.

In ice adsorption experiments, the amounts of the a-PVA and the i-PVA absorbed with each concentration are shown in FIG. 22, wherein the amount of the a-PVA absorbed on the ice surface increases from 16.3% when the concentration is 0.2 mg·mL⁻¹ to 28.7% when the concentration is 1.0 mg·mL⁻¹, and the amount of the a-PVA absorbed on the ice surface is saturated after the concentration is more than 1.0 mg mL⁻¹ with the adsorption amount of about 36.5%. The amount of the i-PVA absorbed on the ice surface is 0%-19.3% when the concentration is less than 1.0 mg·mL⁻¹, and is lower than that of the a-PVA absorbed on the ice surface at the same concentration. At low concentrations, the amounts of the two PVAs adsorbed on the ice surface are not saturated, and the ice surface area covered by the i-PVA is lower than that of the a-PVA.

The amount of the i-PVA adsorbed on the ice surface is higher than that of the a-PVA when the concentration of the i-PVA is more than or equal to 1.2 mg·mL⁻¹, and the amount of the i-PVA absorbed on the ice surface is saturated when the concentration is 2.0 mg·mL⁻¹ with the adsorption amount of 56.5%. Further, it is stated that the amount of the i-PVA required is much greater than that of the a-PVA when the amounts of the two PVAs absorbed on ice surfaces with the same size are saturated. That is, the a-PVA could more effectively cover the surface of ice.

### (2) Assay for ice recrystallization inhibition (IRI) activity

The ice recrystallization inhibition (IRI) activity was assessed using "splat-freezing method", wherein the two PVAs were separately dissolved and dispersed into DPBS solutions, and 10-30 µL of the resulting solution was added dropwise onto the surface of a clean silicon disk pre-cooled at -60 °C at a height of no less than 1.0 m; the solution was slowly heated to -6 °C at a speed of 10 °C·min⁻¹ by using a hot-cold stage, and was annealed for 30 min at this temperature; the sizes of ice crystals were observed and recorded by using a polarizing microscope and a high-speed camera. The hot-cold stage was sealed to ensure that the internal humidity was about 50%. The procedure was repeated at least three times for each sample, and the size of the ice crystal was counted using a Nano Measurer 1.2, with the error of the result being the standard deviation.

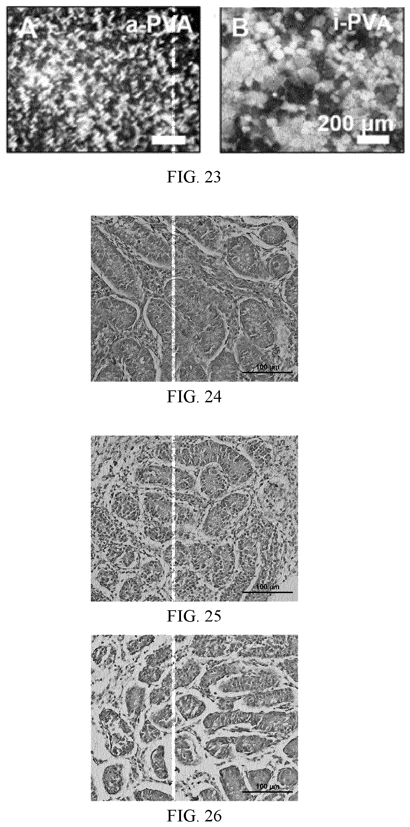

The result is shown in FIG. 23 where the grain size of the a-PVA was significantly smaller than that of the i-PVA at the same concentration, which indicates that the ability of the a-PVA to inhibit the growth of ice crystals is far superior to that of the i-PVA.

According to the results of Example 9, the i-PVA has a weaker affinity for water than the a-PVA. Therefore, the i-PVA tends to exist in an aggregated state in an aqueous solution and on an ice-water interface, while the a-PVA can be well spread in an aqueous solution and on an ice-water interface. The amount of the i-PVA required is much higher than that of the a-PVA when the amounts of the two PVAs absorbed on ice surfaces with the same size are saturated. Therefore, compared with the i-PVA, the a-PVA is a better ice growth inhibition material, playing a better role in inhibiting the growth of ice crystals at lower concentrations.

### C. Formulation of cryopreservation solution and preparation and application embodiments

### [Example 10] Preparation of cryopreservation solution comprising PVA as ice growth inhibition material

### 1. Preparation of cryopreservation solutions: cryopreservation solutions were prepared according to the following formulations.

A cryopreservation solution A comprises the following components per 100 mL:

| Substances | Content |
|---|---|
| PVA (g) | 2.0 |
| Ethylene glycol (mL) | 10 |
| DMSO (mL) | 10 |
| Sucrose (mol·L⁻¹) | 0.5 |
| Fetal bovine serum (mL) | 20 |
| DPBS (mL) | Balance |

Solution preparation steps: 2.0 g of a PVA was dissolved in 25 mL of DPBS in a water bath at 80 °C by heating magnetic stirring, and pH was adjusted to 7.0 to give a solution 1 after the PVA was completely dissolved and cooled to the room temperature; 17 g (0.05 mol) of sucrose (the final concentration of the sucrose in the cryopreservation solution was 0.5 mol·L⁻¹) was ultrasonically dissolved in 25 mL of DPBS, and after the sucrose was completely dissolved, 10 mL of ethylene glycol and 10 mL of DMSO were added to give a solution 2; after returning to room temperature, the solution 1 and the solution 2 were homogeneously mixed, the pH was adjusted, the volume was made up to 80%, and 20 mL of serum was stored separately to be added when the cryopreservation solution was used.

A cryopreservation solution B comprises the following components per 100 mL:

| Substances | Content |
|---|---|
| L-Arg (g) | 8.0 |
| L-Thr (g) | 4.0 |
| PVA (g) | 2.0 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps: 2.0 g of a PVA was dissolved in 20 mL of DPBS in a water bath at 80 °C by heating magnetic stirring, and pH was adjusted to 7.1 to give a solution 1; 8.0 g of L-Arg and 4.0 g of L-Thr were dissolved in 20 mL of DPBS, and the pH was adjusted to 7.1 to give a solution 2; 17 g (0.05 mol) of sucrose (the final concentration of the sucrose in the cryopreservation solution was 0.5 mol·L⁻¹) was ultrasonically dissolved in 20 mL of DPBS, and after the sucrose was completely dissolved, 10 mL of ethylene glycol was added to give a solution 3; after returning to room temperature, the solution 1, the solution 2 and the solution 3 were homogeneously mixed, the pH was adjusted, the volume was made up to 80%, and 20 mL of serum was added when the cryopreservation was used.

A cryopreservation solution C comprises the following components per 100 mL:

| Substances | Content |
|---|---|
| PVA (g) | 2.0 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps: 2.0 g of a PVA was dissolved in 25 mL of DPBS in a water bath at 80 °C by heating magnetic stirring, and pH was adjusted to 6.9 to give a solution 1; 17 g (0.05 mol) of sucrose (the final concentration of the sucrose in the cryopreservation solution was 0.5 mol·L⁻¹) was ultrasonically dissolved in 25 mL of DPBS, and after the sucrose was completely dissolved, 10 mL of ethylene glycol was added to give a solution 2; after returning to room temperature, the solution 1 and the solution 2 were homogeneously mixed, the pH was adjusted, the volume was made up to 80%, and 20 mL of serum was stored separately to be added when the cryopreservation solution was used.

A cryopreservation solution C1 comprises the following components per 100 mL:

| Substances | Content |
|---|---|
| PVA (g) | 1.0 |
| Ethylene glycol (mL) | 10 |
| Sucrose (mol·L⁻¹) | 0.5 |
| Serum (mL) | 20 |
| DPBS (ml) | Balance |

The solution preparation steps were the same as those of the cryopreservation solution C.

A cryopreservation solution D comprises the following components per 100 mL:

| Substances | Content |
|---|---|
| PVA (g) | 2.0 |
| Ethylene glycol (mL) | 10 |
| Sucrose (mol·L⁻¹) | 0.5 |
| DPBS (mL) | Balance |

Solution preparation steps: 2.0 g of a PVA was dissolved in 30 mL of DPBS in a water bath at 80 °C by heating magnetic stirring, and pH was adjusted to 7.0 to give a solution 1; 17 g (0.05 mol) of sucrose (the final concentration of the sucrose in the cryopreservation solution was 0.5 mol·L⁻¹) was ultrasonically dissolved in 25 mL of DPBS, and after the sucrose was completely dissolved, 10 mL of ethylene glycol was added to give a solution 2; after returning to room temperature, the solution 1 and the solution 2 were homogeneously mixed, the pH was adjusted, and the volume was made up to 100 mL for later use.

A cryopreservation solution E comprises the following components per 100 mL:

| Substances | Content |
|---|---|
| Poly-L-proline (g) | 1.5 |
| PVA (g) | 2.0 |
| Ethylene glycol (mL) | 10 |
| Sucrose (mol·L⁻¹) | 0.5 |
| DPBS (ml) | Balance |

Solution preparation steps: 2.0 g of a PVA was dissolved in 25 mL of DPBS in a water bath at 80 °C by heating magnetic stirring, and pH was adjusted to 7.0 to give a solution 1; 1.5 g of poly-L-proline (with the degree of polymerization of 15) was ultrasonically dissolved in another 20 mL of DPBS, and the pH was adjusted to 7.0 to give a solution 2; 17 g (0.05 mol) of sucrose (the final concentration of the sucrose in the cryopreservation solution was 0.5 mol·L⁻¹) was ultrasonically dissolved in 25 mL of DPBS, and after the sucrose was completely dissolved, 10 mL of ethylene glycol was added to give a solution 3; after returning to room temperature, the solution 1, the solution 2 and the solution 3 were homogeneously mixed, the pH was adjusted, and the volume was made up to 100 mL for later use.

The cryopreservation solution F comprises the following components per 100 mL:

| Substances | Content |
|---|---|
| Poly-L-arginine (g, degree of polymerization being 8) | 4.0 |
| PVA (g) | 1.0 |
| Ethylene glycol (mL) | 10 |
| Sucrose (mol·L⁻¹) | 0.5 |
| Serum (mL) | 20 |
| DPBS (ml) | Balance |

The solution preparation steps were the same as those of the cryopreservation solution E, and the serum was added when the cryopreservation solution was used.

2. Preparation of freezing equilibration solutions: the freezing equilibration solutions were prepared according to the following formulations.

A freezing equilibration solution a: 7.5 mL of ethylene glycol and 7.5 mL of DMSO were added to 65 mL of DPBS, and mixed homogeneously, and 20 mL of serum was added when the freezing equilibration solution was used.

A freezing equilibration solution b: 7.5 mL of ethylene glycol was dissolved in 72.5 mL of DPBS, and mixed homogeneously, and 20 mL of serum was added when the freezing equilibration solution was used.

A freezing equilibration solution c: 2.0 g of a PVA was dissolved in 50 mL of DPBS in a water bath at 80 °C by heating magnetic stirring, pH was adjusted to 7.0 after the PVA was completely dissolved, 7.5 mL of ethylene glycol was added, and mixed homogeneously, the pH was adjusted, and the volume was made up to 100 mL for later use.

### Comparative Example:

A freezing equilibration solution a comprises, per 1 mL, 7.5% (v/v) of DMSO, 7.5% (v/v) of ethylene glycol, 20% (v/v) of fetal bovine serum and the balance of DPBS;
A cryopreservation solution 1# comprises, per 1 mL, 15% (v/v) of DMSO, 15% (v/v) of ethylene glycol, 20% (v/v) of fetal bovine serum, 0.5 M sucrose and the balance of DPBS.

A freezing equilibration solution 2# comprises, per 1 mL, 7.5% (v/v) of ethylene glycol, 20% (v/v) of fetal bovine serum and the balance of DPBS;

A cryopreservation solution 2# comprises, per 1 mL, 10% (v/v) of ethylene glycol, 20% (v/v) of fetal bovine serum, 0.5 M sucrose and the balance of DPBS.

A cryopreservation solution 3# comprises, per 1 mL, 10% (v/v) of DMSO, 15% (v/v) of fetal bovine serum and the balance of a-MEM (USA, Invitrogen, C12571500BT).

The three formulations of the thawing solutions used in Example 10 and the comparative examples were as follows:
A thawing solution 1# comprises a thawing solution I (comprising sucrose at 1.0 mol·L⁻¹, 20% of serum and the balance of DPBS), a thawing solution II (comprising sucrose at 0.5 mol·L⁻¹, 20% of serum and the balance of DPBS), a thawing solution III (comprising sucrose at 0.25 mol·L⁻¹, 20% of serum and the balance of DPBS), and a thawing solution IV (comprising 20% of serum and the balance of DPBS).

A thawing solution 2# comprises a thawing solution I (comprising sucrose at 1.0 mol·L⁻¹, a PVA at 20 mg·mL⁻¹ and the balance of DPBS), a thawing solution II (comprising sucrose at 0.5 mol·L⁻¹, a PVA at 20 mg·mL⁻¹ and the balance of DPBS), a thawing solution III (comprising sucrose at 0.25 mol·L⁻¹, a PVA at 20 mg mL⁻¹ and the balance of DPBS), and a thawing solution IV (comprising a PVA at 20 mg·mL⁻¹ and the balance of DPBS).

A thawing solution 3# comprises a thawing solution I (comprising sucrose at 1.0 mol·L⁻¹, a PVA at 20 mg·mL⁻¹, 10 mg·mL⁻¹ polyproline and the balance of DPBS), a thawing solution II (comprising sucrose at 0.5 mol·L⁻¹, a PVA at 20 mg·mL⁻¹, 5.0 mg·mL⁻¹ polyproline and the balance of DPBS), a thawing solution III (comprising sucrose at 0.25 mol·L⁻¹, a PVA at 20 mg·mL⁻¹, 2.5 mg·mL⁻¹ polyproline and the balance of DPBS), and a thawing solution IV (comprising a PVA at 20 mg·mL⁻¹ and the balance of DPBS).

### [Application Example 1]

Oocytes and embryos were cryopreserved using the freezing equilibration solutions and the cryopreservation solutions of the above examples and comparative examples according to the schemes in Table 1 and Table 2, respectively. The survival rates were the average survival rate of 3-12 repeated experiments.

### 1. Cryopreservation of oocytes

Mouse oocytes were firstly equilibrated in a freezing equilibration solution for 5 min, and then put in the prepared cryopreservation solution for 1 min; the equilibrated oocytes in the cryopreservation solution were placed on a straw, then quickly put into liquid nitrogen (-196 °C), and continuously preserved after the carrying rod was sealed; at the time of thawing, the frozen oocytes were equilibrated in the thawing solution I at 37 °C for 5 min, and equilibrated in the thawing solutions II-IV in sequence for 3 min each; and after the thawed oocytes were cultured for 2 h, the number of the survived cells was observed, and the survival rates were calculated (see Table 1).

### 2. Cryopreservation of embryos

Mouse embryos were firstly equilibrated in a freezing equilibration solution for 5 min, and then put into the cryopreservation solution prepared in accordance to the formulations of the above examples and comparative examples for 50 s; the equilibrated embryos in the cryopreservation solution were placed on a straw, then quickly put into liquid nitrogen (-196 °C) and continuously preserved after the carrying rod was sealed; at the time of thawing, the frozen embryos were equilibrated in the thawing solution I at 37 °C for 3 min, and then equilibrated in the thawing solutions II-IV in sequence for 3 min each; and after the thawed embryos were cultured for 2 h, the number of the survived embryos was observed, and the survival rates were calculated (see Table 2).

**Table 1. Survival rates of cryopreserved mouse oocytes**

| No. | Equilibration solution | Cryopreservation solution | Thawing solution | Total number of frozen oocytes | Survival rates after 2 h |
|---|---|---|---|---|---|
| Application Embodiment 1 | a | A | Thawing solution 1# | 67 | 100.0% |
| Application Embodiment 2 | b | B | Thawing solution 1# | 109 | 94.8% |
| Application Embodiment 3 | b | C | Thawing solution 1# | 90 | 97.7% |
| Application Embodiment 4 | c | D | Thawing solution 1# | 50 | 93.4% |
| Application Embodiment 5 | c | D | Thawing solution 2# | 53 | 96.5% |
| Application Embodiment 6 | c | E | Thawing solution 1# | 39 | 89.7% |
| Application Embodiment 7 | c | E | Thawing solution 3# | 60 | 98.6% |
| Comparative Embodiment 1 | a | Freezing solution 1# | Thawing solution 1# | 146 | 95.0% |
| Comparative Embodiment 2 | Equilibration solution 2# | Freezing solution 2# | Thawing solution 1# | 96 | 81.9% |
| Comparative Embodiment 3 | Equilibration solution 2# | Freezing solution 2# | Thawing solution 2# | 44 | 94.7% |

**Table 2. Survival rates of cryopreserved mouse embryos**

| No. | Equilibration solution | Cryopreservation solution | Thawing solution | Total number of frozen embryos | Survival rates after 2 h |
|---|---|---|---|---|---|
| Application Embodiment 8 | c | D | Thawing solution 1# | 41 | 95.8% |
| Application Embodiment 9 | c | E | Thawing solution 1# | 42 | 95.2% |
| Comparative Embodiment 4 | a | Freezing solution 1# | Thawing solution 1# | 38 | 94.3% |
| Comparative Embodiment 5 | Equilibration solution 2# | Freezing solution 2# | Thawing solution 1# | 39 | 82.4% |

The above data indicate that the survival rate of the cryopreservation solution can be no less than 90% and even 100%, and can reach or far exceed a cryopreservation thawing rate of a commercial cryopreservation solution comprising 15% DMSO and commonly available in clinical practice at present, and as can be seen from the comparison of Application Embodiment 1 (comprising 10% DMSO), Comparative Embodiment 2 (comprising 7.5% DMSO) and Comparative Embodiment 1 (namely commercial oocyte cryopreservation solution (comprising 15% DMSO)), the survival rate of oocytes is significantly improved by adding PVA; Application Embodiments 2-3 also show that the cryopreservation solution can have higher survival rates of oocytes or embryos by adding a small amount of DMSO or not adding DMSO, solving the problem that the DMSO concentration of commercial cryopreservation solutions commonly available in clinical practice is high and the damage to cells is large; moreover, Application Embodiments 5 and 7-9 show that higher survival rates of oocytes or embryos can be realized under the condition that DMSO and serum are not added in the freezing solutions, equilibration solutions and thawing solutions. The DMSO-free or serum-free cryopreservation solution solves the problems of short shelf life, introduction of parasitic biological pollutants and the like caused by serum comprised in the commercial cryopreservation solutions commonly available in clinical practice at present.

### [Application Example 2] Cryopreservation of human umbilical cord mesenchymal stem cells

Human umbilical cord mesenchymal stem cells were cryopreserved using the cryopreservation solutions of the above examples and comparative examples according to the scheme in Table 3.

Cryopreservation of human umbilical cord mesenchymal stem cells by microdroplet method: digesting human umbilical cord mesenchymal stem cells on a culture dish using 25% pancreatin for 2 min, putting the digested human umbilical cord mesenchymal stem cells into a culture solution (10% FBS + a-MEM culture medium) of the same volume, gently pipetting until the stem cells completely fall off, adding the cells into a 1.5 mL centrifuge tube for centrifuging for 5 min at 1000 rmp, discarding the supernatant (separating the cells from the culture medium), adding 10 µL of a freezing solution to the bottom of the centrifuge tube, gently pipetting to disperse stem cell clusters, placing the 10 µL of the freezing solution with the stem cells on a freezing slide, and cryopreserving the solution into liquid nitrogen (-196 °C). At the time of thawing, the straw with the cells and the freezing solution was placed directly in a culture medium at 37 °C for thawing. After thawing, cells were stained with trypan blue to observe the survival rates, and the number of cells was counted using an instrument JIMBIO-FIL, survival rate = number of live cells/total number of cells (see Table 3).

**Table 3. Survival rates of cryopreserved human umbilical cord mesenchymal stem cells**

| No. | Cryopreservation solution | Cryopreservation method | Survival rates |
|---|---|---|---|
| Application Embodiment 10 | C1 | Microdroplet method | 72.2% |
| Application Embodiment 11 | D | Microdroplet method | 77.1% |
| Application Embodiment 12 | F | Microdroplet method | 92.4% |
| Comparative Embodiment 6 | Freezing solution 1# | Microdroplet method | 63.9% |
| Comparative Embodiment 7 | Freezing solution 3# | Microdroplet method | 76.6% |

When the cryopreservation solution disclosed herein is used for cryopreservation of the human umbilical cord mesenchymal stem cells, the survival rates of the stem cells can reach 92.4% and 72.2%, respectively (in Application Embodiments 12 and 10) although no DMSO is added, and the survival rate can even reach 77.1% when no DMSO and serum is added, reaching the survival level of the existing freezing regent. This means that the freezing reagent can have the same effectiveness in freezing stem cells as a conventional freezing solution, a cryopreservation thawing rate of the reagent can reach or even be far higher than that of a commonly available cryopreservation solution comprising 10% DMSO (in Comparative Embodiment 7), and the PVA-based cryopreservation effect is remarkably superior to the PVA-free cryopreservation effect in Comparative Embodiment 6.

### [Application Example 3] Cryopreservation of ovarian organs and ovarian tissues

The ovarian organs of mice newly born within 3 days and the ovarian tissue slices of sexually mature mice were cryopreserved using the freezing equilibration solutions and cryopreservation solutions of the above examples and comparative examples according to the schemes in Table 4 and Table 5.

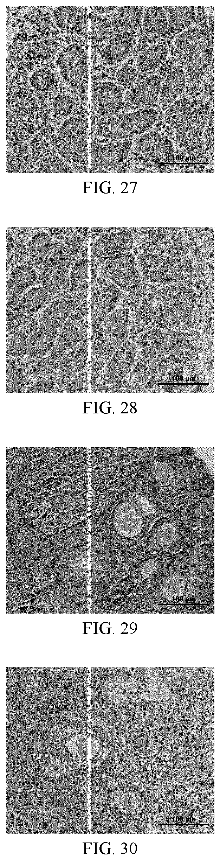

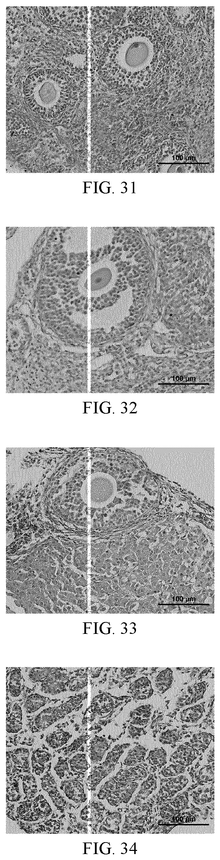

The intact ovarian organs or ovarian tissue slices were firstly equilibrated in an equilibration solution at room temperature for 25 min, and then put into the prepared cryopreservation solution for 15 min. Next, the intact ovarian organs or ovarian tissue slices were placed on a straw and put into liquid nitrogen for preservation. After being thawed, the intact ovarian organs or ovarian tissue slices were put into a culture solution (10% FBS + a-MEM) in an incubator at 37 °C in the presence of 5% CO₂ for 2 h for further thawing. Next, the intact ovarian organs or ovarian tissue slices were fixed with 4% paraformaldehyde, embedded in paraffin, and subjected to HE staining, and then the morphology was observed. The results are shown in FIGs. 24-33, wherein FIG. 24 is a picture of a slice of a fresh unfrozen ovarian organ, and FIG. 29 is a picture of a slice of fresh unfrozen ovarian tissue.

**Table 4. Ovarian organ cryopreservation scheme**

| No. | Equilibration solution | Cryopreservation solution | Thawing solution | Morphology |
|---|---|---|---|---|
| Application Embodiment 13 | c | D | Thawing solution 2# | FIG. 26 |
| Application Embodiment 14 | b | C1 | Thawing solution 1# | FIG. 27 |
| Application Embodiment 15 | b | F | Thawing solution 1# | FIG. 28 |
| Comparative Embodiment 8 | a | Freezing solution 1# | Thawing solution 1# | FIG. 25 |

**Table 5. Ovarian tissue cryopreservation scheme**

| No. | Equilibration solution | Cryopreservation solution | Thawing solution | Morphology |
|---|---|---|---|---|
| Application Embodiment 16 | c | D | Thawing solution 2# | FIG. 31 |
| Application Embodiment 17 | b | C1 | Thawing solution 1# | FIG. 32 |
| Application Embodiment 18 | b | F | Thawing solution 1# | FIG. 33 |
| Comparative Embodiment 9 | a | Freezing solution 1# | Thawing solution 1# | FIG. 30 |

As can be seen from FIGs. 24-28, compared with Comparative Embodiment 8 free of polyvinyl alcohol and fresh unfrozen ovarian organs, the schemes of Application Embodiments 13-15 are characterized in that: the original follicle structure is relatively intact, the interstitial structure is relatively intact, the cytoplasm of cells is relatively homogeneous and lightly stained in a relatively large amount, and nucleus shrinkage and deep staining were relatively mild; blood vessels have intact vessel wall structures and less luminal collapse, the cytoplasm of endothelial cells is relatively homogeneous and lightly stained in a relatively large amount, and nucleus shrinkage and deep staining were relatively mild. As can be seen, Application Embodiments 13-15 have better cryopreservation effects on ovarian organs.

As can be seen from FIGs. 29-33, compared with Comparative Embodiment 9 and fresh unfrozen ovarian tissues, the schemes of Application Embodiments 16-18 are characterized in that: the antral follicle structure is relatively intact, the interstitial structure is relatively intact, the cytoplasm is relatively homogeneous and lightly stained in a relatively large amount, and nucleus shrinkage and deep staining were relatively mild. It can be seen that the cryopreservation solution disclosed herein has a better cryopreservation effect on ovarian tissues than the prior art.

It can be seen that the cryopreservation solution prepared with the biomimetic PVA-based ice growth inhibition material as a main component disclosed herein has a good inhibition effect on the growth of ice crystals, can be used with DMSO being reduced in the preservation system or even without DMSO, maintain good biocompatibility, and can be simultaneously applied to cryopreservation of oocytes, embryos, stem cells, reproductive organs and tissues where high cell survival rates and good biological activity can be achieved.

### [Example 11] Preparation of cryopreservation solution comprising amino acid as ice growth inhibition material

A cryopreservation solution G comprises the following components per 100 mL:

| Substances | Content |
|---|---|
| L-Arg (g) | 16.0 |
| L-Thr (g) | 8.0 |
| DMSO (mL) | 10 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps (total volume: 100 mL): 16 g of L-Arg and 8 g of L-Thr were dissolved in 25 mL of DPBS, and pH was adjusted to 6.9 to give a solution 1; 17 g (0.05 mol) of sucrose (the final concentration of the sucrose in the cryopreservation solution was 0.5 mol·L⁻¹) was ultrasonically dissolved in 25 mL of DPBS, and after the sucrose was completely dissolved, 10 mL of ethylene glycol and 10 mL of DMSO were sequentially added to give a solution 2; after returning to room temperature, the solution 1 and the solution 2 were homogeneously mixed, the pH was adjusted to 6.9, the volume was made up to 80% with DPBS, and 20 mL of fetal bovine serum was stored separately to be added before the cryopreservation solution was used.

A cryopreservation solution H comprises the following components per 100 mL:

| Substances | Content |
|---|---|
| Poly-L-proline (g, degree of polymerization being 15) | 1.5 |
| DMSO (mL) | 10 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps: 1.5 g of poly-L-proline (with the degree of polymerization of 15) was ultrasonically dissolved in 25 mL of DPBS, and pH was adjusted to 6.8 to give a solution 1; 17 g (0.05 mol) of sucrose was ultrasonically dissolved in 25 mL of DPBS, and after the sucrose was completely dissolved, 10 mL of ethylene glycol and 10 mL of DMSO were sequentially added to give a solution 2; after returning to room temperature, the solution 1 and the solution 2 were homogeneously mixed, the pH was adjusted to 7.0, the volume was made up to 80% with DPBS, and 20 mL of serum was stored separately to be added before the cryopreservation solution was used.

A cryopreservation solution I comprises the following components per 100 mL:

| Substances | Content |
|---|---|
| Poly-L-arginine (g, degree of polymerization being 8) | 1.5 |
| DMSO (mL) | 10 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps (total volume: 100 mL): 1.5 g of poly-L-arginine (with the degree of polymerization of 8) was ultrasonically dissolved in 25 mL of DPBS, and pH was adjusted to 7.0 to give a solution 1; 17 g (0.05 mol) of sucrose was ultrasonically dissolved in 20 mL of DPBS, and after the sucrose was completely dissolved, 10 mL of ethylene glycol and 10 mL of DMSO were sequentially added to give a solution 2; after returning to room temperature, the solution 1 and the solution 2 were homogeneously mixed, the pH was adjusted to 7.0, the volume was made up to 80% with DPBS, and 20 mL of serum was stored separately to be added before the cryopreservation solution was used.

A cryopreservation solution J comprises the following components per 100 mL:

| Substances | Content |
|---|---|
| Poly-L-arginine (g, degree of polymerization being 8) | 4.0 |
| DMSO (mL) | 7.5 |
| Ethylene glycol (mL) | 10 |
| Sucrose (mol·L⁻¹) | 0.5 |
| Fetal bovine serum (mL) | 20 |
| DPBS (mL) | Balance |

The solution preparation steps were the same as those of the cryopreservation solution I.

A cryopreservation solution K comprises the following components per 100 mL:

| Substances | Content |
|---|---|
| Poly-L-proline (g, degree of polymerization being 8) | 4.0 |
| DMSO (mL) | 7.5 |
| Ethylene glycol (mL) | 10 |
| Sucrose (mol·L⁻¹) | 0.5 |
| Fetal bovine serum (mL) | 20 |
| DPBS (mL) | Balance |

The solution preparation steps were the same as those of the cryopreservation solution I.

A cryopreservation solution L comprises the following components per 100 mL:

| Substances | Content |
|---|---|
| L-Arg (g) | 16.0 |
| L-Thr (g) | 8.0 |
| DMSO (mL) | 7.5 |
| Ethylene glycol (mL) | 10 |
| Sucrose (mol·L⁻¹) | 0.5 |
| Fetal bovine serum (mL) | 20 |
| DPBS (mL) | Balance |

The solution preparation steps were the same as those of the cryopreservation solution G.

Preparation of freezing equilibration solutions: the freezing equilibration solutions were prepared according to the following formulations.

A freezing equilibration solution a: 7.5 mL of ethylene glycol and 7.5 mL of DMSO were added to 65 mL of DPBS, and mixed homogeneously, and 20 mL of serum was added when the freezing equilibration solution was used.

A freezing equilibration solution b: 7.5 mL of ethylene glycol was added to 72.5 mL of DPBS, and mixed homogeneously, and 20 mL of serum was added when the freezing equilibration solution was used.

### Comparative Example 2:

A freezing equilibration solution a comprises, per 1 mL, 7.5% (v/v) of DMSO, 7.5% (v/v) of ethylene glycol, 20% (v/v) of fetal bovine serum and the balance of DPBS;
A cryopreservation solution 1# comprises, per 1 mL, 15% (v/v) of DMSO, 15% (v/v) of ethylene glycol, 20% (v/v) of fetal bovine serum, 0.5 M sucrose and the balance of DPBS.

A cryopreservation solution 3# comprises, per 1 mL, 10% (v/v) of DMSO, 15% (v/v) of fetal bovine serum and the balance of a-MEM (USA, Invitrogen, C12571500BT).

The formulation of the thawing solutions used in Example 11 and Comparative Example 2 was as follows:
A thawing solution 1# comprises a thawing solution I (comprising sucrose at 1.0mol·L⁻¹, 20% of serum and the balance of DPBS), a thawing solution II (comprising sucrose at 0.5 mol·L⁻¹, 20% of serum and the balance of DPBS), a thawing solution III (comprising sucrose at 0.25 mol·L⁻¹, 20% of serum and the balance of DPBS), and a thawing solution IV (comprising 20% of serum and the balance of DPBS).

### [Application Example 4] Cryopreservation of oocytes and embryos

Oocytes and embryos were cryopreserved using the freezing equilibration solutions and cryopreservation solutions of Example 11 and Comparative Example 2 according to the schemes in Table 6 and Table 7. The freezing and thawing methods were the same as those in Application Example 1.

**Table 6. Survival rates of cryopreserved mouse oocytes**

| No. | Equilibration solution | Cryopreservation solution | Thawing solution | Total number of frozen oocytes | Survival rates after 2 h |
|---|---|---|---|---|---|
| Application Embodiment 19 | a | G | Thawing solution 1# | 67 | 98.5% |
| Application Embodiment 20 | a | H | Thawing solution 1# | 109 | 96.3% |
| Application Embodiment 21 | a | I | Thawing solution 1# | 67 | 95.5% |
| Comparative Embodiment 10 | a | Freezing solution 1# | Thawing solution 1# | 146 | 95.0% |

**Table 7. Survival rates of cryopreserved mouse embryos**

| No. | Equilibration solution | Cryopreservation solution | Thawing solution | Total number of frozen embryos | Survival rates after 2 h |
|---|---|---|---|---|---|
| Application Embodiment 22 | a | J | Thawing solution 1# | 25 | 100.00 % |
| Comparative Embodiment 11 | a | Freezing solution 1# | Thawing solution 1# | 38 | 94.30% |

As can be seen from the data in Tables 6 and 7, when the cryopreservation solution disclosed herein is used for cryopreservation of oocytes and embryos after the amount of DMSO and EG is reduced, the survival rate of the oocytes can reach no less than 95%, the survival rate of the embryos can reach 100%, a cryopreservation thawing rate of the cryopreservation solution disclosed herein can reach or even be far higher than that of a commercial cryopreservation solution comprising 15% DMSO and commonly available in clinical practice at present (in Comparative Embodiments 10-11), and the cryopreservation effect with the addition of the biomimetic amino acid ice growth inhibition material is remarkably superior to the cryopreservation effect without the addition of the biomimetic amino acid ice growth inhibition material.

### [Application Example 5] Cryopreservation of human umbilical cord mesenchymal stem cells

Human umbilical cord mesenchymal stem cells were cryopreserved using the cryopreservation solutions of Example 11 and Comparative Example 2 according to the scheme in Table 8. Freezing and thawing methods are seen from Application Example 2.

**Table 8. Survival rates of cryopreserved human umbilical cord mesenchymal stem cells**

| No. | Cryopreservation solution | Cryopreservation method | Survival rates |
|---|---|---|---|
| Application Embodiment 23 | J | Microdroplet method | 81.2% |
| Application Embodiment 24 | K | Microdroplet method | 82.6% |
| Application Embodiment 25 | L | Microdroplet method | 80.5% |
| Comparative Embodiment 12 | Freezing solution 1# | Microdroplet method | 63.9% |
| Comparative Embodiment 13 | Freezing solution 3# | Microdroplet method | 76.6% |

When the cryopreservation solution disclosed herein is used for cryopreservation of human umbilical cord mesenchymal stem cells, the survival rate of the stem cells can reach no less than 80% by adding only a small amount of DMSO (7.5%) or even not adding DMSO (for example, in Application Embodiments 23-25). This means that the freezing reagent can not only have the same effectiveness in freezing stem cells as a conventional cryopreservation solution, but also has a cryopreservation thawing rate even far higher than that of a commonly available cryopreservation solution comprising 10% DMSO (in Comparative Embodiment 13), and the cryopreservation effect with the addition of the biomimetic amino acid ice growth inhibition material is remarkably superior to the cryopreservation effect without the addition of the biomimetic amino acid ice growth inhibition material (in Comparative Embodiments 14 and 15).

### [Application Example 6] Cryopreservation of ovarian organs and ovarian tissues

The ovarian organs of mice newly born within 3 days and the ovarian tissue slices of sexually mature mice were cryopreserved using the freezing equilibration solutions and cryopreservation solutions of Example 11 and Comparative Example 2 according to the schemes in Table 9 and Table 10. Methods for freezing and thawing ovarian organs and ovarian tissues of sexually mature mice are seen from Application Example 3.

**Table 9. Ovarian organ cryopreservation scheme**

| No. | Equilibration solution | Cryopreservation solution | Thawing solution | Morphology |
|---|---|---|---|---|
| Application Embodiment 26 | a | J | Thawing solution 1# | FIG. 34 |
| Application Embodiment 27 | a | L | Thawing solution 1# | FIG. 35 |
| Application Embodiment 28 | a | K | Thawing solution 1# | FIG. 36 |
| Comparative Embodiment 14 | a | Freezing solution 1# | Thawing solution 1# | FIG. 25 |

**Table 10. Ovarian tissue cryopreservation scheme**

| No. | Equilibration solution | Cryopreservation solution | Thawing solution | Morphology |
|---|---|---|---|---|
| Application Embodiment 29 | a | J | Thawing solution 1# | FIG. 37 |
| Application Embodiment 30 | a | L | Thawing solution 1# | FIG. 38 |
| Application Embodiment 31 | a | K | Thawing solution 1# | FIG. 39 |
| Comparative Embodiment 15 | a | Freezing solution 1# | Thawing solution 1# | FIG. 30 |

### [Example 11] Preparation of cryopreservation solution comprising peptidic compound as ice growth inhibition material

A cryopreservation solution M comprises the following components per 100 mL:

| Substances | Content |
|---|---|
| TR (g) | 28 |
| DMSO (mL) | 7.5 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps (total volume: 100 mL): 28 g of TR was ultrasonically dissolved in 25 mL of DPBS, and pH was adjusted to 7.0 to give a solution 1; 0.05 mol of sucrose was ultrasonically dissolved in 25 mL of DPBS, and after the sucrose was completely dissolved, 10 mL of ethylene glycol and 7.5 mL of DMSO were sequentially added to give a solution 2; after returning to room temperature, the solution 1 and the solution 2 were homogeneously mixed, the pH was adjusted, the volume was made up to 80% with DPBS, and finally 20 mL of serum was added before the cryopreservation solution was used.

A cryopreservation solution N comprises the following components per 100 mL:

| Substances | Content |
|---|---|
| TPT (g) | 28 |
| DMSO (mL) | 7.5 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps (total volume: 100 mL): 28 g of TPT was ultrasonically dissolved in 25 mL of DPBS, and pH was adjusted to 7.0 to give a solution 1; 0.05 mol of sucrose was ultrasonically dissolved in 25 mL of DPBS, and after the sucrose was completely dissolved, 10 mL of ethylene glycol and 7.5 mL of DMSO were sequentially added to give a solution 2; after returning to room temperature, the solution 1 and the solution 2 were homogeneously mixed, the pH was adjusted, the volume was made up to 80% with DPBS, and finally 20 mL of serum was added before the cryopreservation solution was used.

A cryopreservation solution O comprises the following components per 100 mL:

| Substances | Content |
|---|---|
| TR (g) | 28 |
| Ethylene glycol (mL) | 10 |
| Sucrose (mol·L⁻¹) | 0.5 |
| Fetal bovine serum (mL) | 20 |
| DPBS (mL) | Balance |

Solution preparation steps (total volume: 100 mL): 28 g of TR was ultrasonically dissolved in 25 mL of DPBS, and pH was adjusted to 7.0 to give a solution 1; 0.05 mol of sucrose was ultrasonically dissolved in 25 mL of DPBS, and after the sucrose was completely dissolved, 10 mL of ethylene glycol was added to give a solution 2; after returning to room temperature, the solution 1 and the solution 2 were homogeneously mixed, the pH was adjusted, the volume was made up to 80% with DPBS, and finally 20 mL of serum was added before the cryopreservation solution was used.

Preparation of freezing equilibration solutions: the freezing equilibration solutions were prepared according to the following formulations.

A freezing equilibration solution a: 7.5 mL of ethylene glycol and 7.5 mL of DMSO were added to 65 mL of DPBS, and mixed homogeneously, and 20 mL of serum was added when the freezing equilibration solution was used.

### Comparative Example 3:

A freezing equilibration solution a comprises, per 1 mL, 7.5% (v/v) of DMSO, 7.5% (v/v) of ethylene glycol, 20% (v/v) of fetal bovine serum and the balance of DPBS;

A cryopreservation solution 1# comprises, per 1 mL, 15% (v/v) of DMSO, 15% (v/v) of ethylene glycol, 20% (v/v) of fetal bovine serum, 0.5 M sucrose and the balance of DPBS.

A cryopreservation solution 3# comprises, per 1 mL, 10% (v/v) of DMSO, 15% (v/v) of fetal bovine serum and the balance of a-MEM (USA, Invitrogen, C12571500BT).

The formulation of the thawing solutions used in Example 12 and Comparative Example 3 was as follows:
A thawing solution 1# comprises a thawing solution I (comprising sucrose at 1.0mol·L⁻¹, 20% of serum and the balance of DPBS), a thawing solution II (comprising sucrose at 0.5 mol·L⁻¹, 20% of serum and the balance of DPBS), a thawing solution III (comprising sucrose at 0.25 mol·L⁻¹, 20% of serum and the balance of DPBS), and a thawing solution IV (comprising 20% of serum and the balance of DPBS).

### [Application Example 7] Cryopreservation of oocytes and embryos

Oocytes and embryos were cryopreserved using the freezing equilibration solutions and cryopreservation solutions of Example 13 and Comparative Example 2 according to the schemes in Table 11 and Table 12. The freezing and thawing methods were the same as those in Application Example 1.

**Table 11. Survival rates of cryopreserved mouse oocytes**

| No. | Equilibration solution | Freezing solution | Thawing solution | Total number of frozen oocytes | Survival rates after 2 h |
|---|---|---|---|---|---|
| Application Embodiment 32 | a | M | Thawing solution 1# | 93 | 96.2% |
| Application Embodiment 33 | a | N | Thawing solution 1# | 48 | 90% |
| Comparative Embodiment 16 | a | Freezing solution 1# | Thawing solution 1# | 146 | 95% |

**Table 12. Survival rates of cryopreserved mouse embryos**

| No. | Equilibration solution | Freezing solution | Thawing solution | Total number of embryos | Survival rates after 2 h |
|---|---|---|---|---|---|
| Application Embodiment 34 | a | M | Thawing solution 1# | 41 | 95.9% |
| Comparative Embodiment 17 | a | Freezing solution 1# | Thawing solution 1# | 38 | 94.3% |

The data in Tables 11 and 12 show that the polypeptides disclosed herein are used for cryopreservation of oocytes and embryos, and that the survival rates of oocytes and embryos of the existing commercial cryopreservation solution (DMSO content 15%) can be achieved by adding only a small amount of DMSO (7.5%), and the data of Application Embodiments 32 and 34 show that TR polypeptides have a more excellent cryopreservation effect on oocytes and embryos.

### [Application Example 8] Cryopreservation of human umbilical cord mesenchymal stem cells

Human umbilical cord mesenchymal stem cells were cryopreserved using the cryopreservation solutions of Example 12 and Comparative Example 3 according to the scheme in Table 13. Freezing and thawing methods are seen from Application Example 2.

**Table 13. Survival rates of cryopreserved human umbilical cord mesenchymal stem cells**

| No. | Cryopreservation solution | Cryopreservation method | Survival rates |
|---|---|---|---|
| Application Embodiment 35 | M | Microdroplet method | 87.8% |
| Application Embodiment 36 | O | Microdroplet method | 75.1% |
| Comparative Embodiment 18 | Freezing solution 3# | Microdroplet method | 76.6% |

According to the results in Table 13, the cryopreservation solution disclosed herein without adding DMSO or adding only a small amount of DMSO (7.5%) can have a cell survival rate equivalent to that of a cryopreservation solution comprising 10% DMSO in the prior art, so that the amount of DMSO is greatly reduced, the damage and toxicity of DMSO to cells are reduced, and the passage stability and cell activity of the frozen stem cells can be greatly improved.

### [Application Example 9] Cryopreservation of ovarian organs and ovarian tissues

The ovarian organs of mice newly born within 3 days and the ovarian tissue slices of sexually mature mice were cryopreserved using the freezing equilibration solutions and cryopreservation solutions of Example 12 and Comparative Example 3 according to the schemes in Table 14 and Table 15. Methods for freezing and thawing ovarian organs and ovarian tissues of sexually mature mice are seen from Application Example 3.

**Table 14. Ovarian organ cryopreservation scheme**

| No. | Equilibration solution | Cryopreservation solution | Thawing solution | Morphology |
|---|---|---|---|---|
| Application Embodiment 37 | a | M | Thawing solution 1# | FIG. 40 |
| Comparative Embodiment 19 | a | Freezing solution 1# | Thawing solution 1# | FIG. 25 |

**Table 15. Ovarian tissue cryopreservation scheme**

| No. | Equilibration solution | Cryopreservation solution | Thawing solution | Morphology |
|---|---|---|---|---|
| Application Embodiment 38 | a | M | Thawing solution 1# | FIG. 41 |
| Comparative Embodiment 20 | a | Freezing solution 1# | Thawing solution 1# | FIG. 30 |

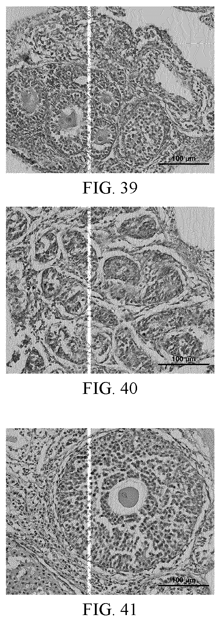

As can be seen from FIGs. 24, 25 and 40, compared with the comparative embodiments free of the biomimetic peptide ice growth inhibition material (FIGs 25 and 30), a picture of a slice of the thawed ovarian organ cryopreserved in the cryopreservation solution of Application Embodiment 37 shows that the follicle structure is relatively intact, the interstitial structure is relatively intact, the cytoplasm is relatively homogeneous and lightly stained in a relatively large amount, and nucleus shrinkage and deep staining were relatively mild; and blood vessels have intact vessel wall structures and less luminal collapse, the cytoplasm of endothelial cells is relatively homogeneous and lightly stained in a relatively large amount, and nucleus shrinkage and deep staining were relatively mild. As can be seen, Application Embodiment 37 has a better cryopreservation effect on ovarian organs.

As can be seen from FIGs. 29, 30 and 41, compared with fresh unfrozen ovarian tissues of mature mice of Comparative Embodiment 22, the scheme of Application Embodiment 38 is characterized in that: the structures of follicles in the growth phase and antral follicles are relatively intact. It can be seen that the cryopreservation solution disclosed herein has a better cryopreservation effect on ovarian tissues than the prior art.

It can be seen that the cryopreservation solution prepared with the biomimetic peptide ice growth inhibition material as a main component disclosed herein can be simultaneously applied to cryopreservation of oocytes, embryos, stem cells, reproductive organs and tissues, where high cell survival rates and good biological activity can be achieved.

The examples of the present invention have been described above. Additionally, the above examples describe compounds, their preparation and their use but the compounds, their preparation or their use do not form part of the claimed invention. The invention is defined in the appended claims.

## Claims

1. A molecular design method for an ice growth inhibition material, comprising the following steps:
(1) constructing a library for structures of compound molecules, wherein the compound molecules comprise a hydrophilic group and an ice-philic group;
(2) simulating and evaluating the spreading performance of each of the compound molecules at an ice-water interface by adopting molecular dynamics (MD) simulation on a computer; and
(3) screening the compound molecules with desired affinities for ice and water;
Wherein in the MD simulation of the step (2), simulation and calculation are performed on interactions between the compound molecules, interactions between the compound molecules and the water molecules, and interactions between the compound molecules and ice-water molecules;
Wherein the interactions include the formation of a hydrogen bond, a Van der Waals interaction, an electrostatic interaction, a hydrophobic interaction or a π-π interaction.

2. The molecular design method according to claim 1, wherein the MD simulation of the step (2) is performed by GROMACS, AMBER, CHARMM, NAMD, or LAMMPS.

3. The molecular design method according to claim 1, wherein
in the MD simulation of the step (2), a model of a water molecule is selected from models of TIP3P, TIP4P, TIP4P/2005, SPC, TIP3P, TIP5P and SPC/E, preferably TIP4P/2005 model of a water molecule; and/or
in the MD simulation of the step (2), a force field parameter is provided by one of GROMOS, ESFF, MM force field, AMBER, CHARMM, COMPASS, UFF, CVFF and other force fields; and/or
in the MD simulation of the step (2), a temperature and pressure are adjusted when the simulation and calculation are performed on the interactions between the molecules; preferably, the temperature and the pressure are adjusted by using a V-rescale temperature regulator and a pressure regulator; and/or
in the MD simulation of the step (2), a molecular configuration of the compound molecules is maintained by selecting a potential energy parameter; and/or
in the step (2), periodic boundary conditions are adopted for x-direction, y-direction and z-direction when an aqueous solution system is simulated; periodic boundary conditions are adopted for x-direction and y-direction when an ice-water mixed system is simulated; and/or
in the MD simulation of the step (2), a cubic or octahedral box of water is selected; and/or
a main chain of the compound molecules is a carbon chain or peptide chain structure; and/or
the hydrophilic group is a functional group capable of forming a non-covalent interaction with a water molecule; and/or
the ice-philic group is a functional group capable of forming a non-covalent interaction with ice; and/or
the ice growth inhibition material is formed by covalently bonding a block comprising a hydrophilic group to a block comprising an ice-philic group, or is formed by ionically bonding a molecule comprising a hydrophilic group to a molecule comprising an ice-philic group.

4. The molecular design method according to claim 1, wherein
in the MD simulation of the step (2), a cubic box of water with dimensions of 3.9 × 3.6 × 1.0 nm³ is selected; and/or
the hydrophilic group is a functional group capable of forming a hydrogen bond, a Van der Waals interaction,
an electrostatic interaction, a hydrophobic interaction or a π-π interaction with water; and/or
the ice-philic group is a functional group capable of forming a hydrogen bond, a Van der Waals interaction, an electrostatic interaction, a hydrophobic interaction or a π-π interaction with ice.

5. The molecular design method according to claim 4, wherein
the hydrophilic group is selected from at least one of hydroxyl (-OH), amino (-NH₂), carboxyl (-COOH) and amido (-CONH₂), or from a compound molecule, such as a hydrophilic amino acid such as proline (L-Pro), arginine (L-Are) and lysine (L-Lys), glucono delta-lactone (GDL) and a saccharide; and/or
the ice-philic group is selected from hydroxyl (-OH), amino (-NH₂), phenyl (-C₆H₅), pyrrolidinyl (-C₄H₈N) or from a compound molecule, such as an ice-philic amino acid such as glutamine (L-Gln), threonine (L-Thr) and aspartic acid (L-Asn), a benzene ring (C₆H₆) and pyrrolidine (C₄H₉N).

## Patentansprüche

1. Verfahren zur Molekülgestaltung für ein Material zur Eiswachstumshemmung, umfassend die folgenden Schritte:
(1) Konstruieren einer Bibliothek für Strukturen von Verbindungsmolekülen, wobei die Verbindungsmoleküle eine hydrophile Gruppe und eine eisphile Gruppe umfassen;
(2) Simulieren und Bewerten der Ausbreitungsleistung jedes der Verbindungsmoleküle an einer Eis-Wasser-Grenzfläche durch Anwendung einer Molekulardynamik-Simulation (MD-Simulation) auf einem Computer; und
(3) Auswählen der Verbindungsmoleküle mit gewünschten Affinitäten zu Eis und Wasser;
wobei in der MD-Simulation des Schritts (2) Simulation und Berechnung von Wechselwirkungen zwischen den Verbindungsmolekülen, Wechselwirkungen zwischen den Verbindungsmolekülen und den Wassermolekülen sowie Wechselwirkungen zwischen den Verbindungsmolekülen und Eis-Wasser-Molekülen durchgeführt werden;
wobei die Wechselwirkungen die Ausbildung einer Wasserstoffbindung, einer Van-der-Waals-Wechselwirkung, einer elektrostatischen Wechselwirkung, einer hydrophoben Wechselwirkung oder einer π-π-Wechselwirkung umfassen.

2. Verfahren zur Molekülgestaltung nach Anspruch 1, wobei die MD-Simulation des Schritts (2) mittels GROMACS, AMBER, CHARMM, NAMD oder LAMMPS durchgeführt wird.

3. Verfahren zur Molekülgestaltung nach Anspruch 1, wobei
in der MD-Simulation des Schritts (2) ein Modell eines Wassermoleküls aus Modellen TIP3P, TIP4P, TIP4P/2005, SPC, TIP3P, TIP5P und SPC/E ausgewählt wird, vorzugsweise das TIP4P/2005-Modell eines Wassermoleküls; und/oder
in der MD-Simulation des Schritts (2) ein Kraftfeldparameter durch eines von GROMOS, ESFF, MM-Kraftfeld, AMBER, CHARMM, COMPASS, UFF, CVFF und anderen Kraftfeldern bereitgestellt wird; und/oder
in der MD-Simulation des Schritts (2) eine Temperatur und ein Druck angepasst werden, wenn die Simulation und Berechnung der Wechselwirkungen zwischen den Molekülen durchgeführt werden; vorzugsweise die Temperatur und der Druck unter Verwendung eines V-rescale-Temperaturreglers und eines Druckreglers angepasst werden; und/oder
in der MD-Simulation des Schritts (2) eine Molekülkonfiguration der Verbindungsmoleküle durch Auswahl eines potentiellen Energieparameters aufrechterhalten wird; und/oder
im Schritt (2) periodische Randbedingungen für x-Richtung, y-Richtung und z-Richtung angewendet werden, wenn ein wässriges Lösungssystem simuliert wird; periodische Randbedingungen für x-Richtung und y-Richtung angewendet werden, wenn ein Eis-Wasser-Mischsystem simuliert wird; und/oder
in der MD-Simulation des Schritts (2) ein kubischer oder oktaedrischer Wasserkasten ausgewählt wird; und/oder
eine Hauptkette der Verbindungsmoleküle eine Kohlenstoffketten- oder Peptidkettenstruktur ist; und/oder
die hydrophile Gruppe eine funktionelle Gruppe ist, die fähig ist, eine nichtkovalente Wechselwirkung mit einem Wassermolekül auszubilden; und/oder
die eisphile Gruppe eine funktionelle Gruppe ist, die fähig ist, eine nichtkovalente Wechselwirkung mit Eis auszubilden; und/oder
das Material zur Eiswachstumshemmung durch kovalentes Binden eines Blocks, der eine hydrophile Gruppe umfasst, an einen Block, der eine eisphile Gruppe umfasst, gebildet ist oder durch ionisches Binden eines Moleküls, das eine hydrophile Gruppe umfasst, an ein Molekül, das eine eisphile Gruppe umfasst, gebildet ist.

4. Verfahren zur Molekülgestaltung nach Anspruch 1, wobei
in der MD-Simulation des Schritts (2) ein kubischer Wasserkasten mit Abmessungen von 3,9 × 3,6 × 1,0 nm³ ausgewählt wird; und/oder
die hydrophile Gruppe eine funktionelle Gruppe ist, die fähig ist, mit Wasser eine Wasserstoffbindung, eine Van-der-Waals-Wechselwirkung, eine elektrostatische Wechselwirkung, eine hydrophobe Wechselwirkung oder eine π-π-Wechselwirkung auszubilden; und/oder
die eisphile Gruppe eine funktionelle Gruppe ist, die fähig ist, mit Eis eine Wasserstoffbindung, eine Van-der-Waals-Wechselwirkung, eine elektrostatische Wechselwirkung, eine hydrophobe Wechselwirkung oder eine π-π-Wechselwirkung auszubilden.

5. Verfahren zur Molekülgestaltung nach Anspruch 4, wobei
die hydrophile Gruppe aus mindestens einem von Hydroxyl (-OH), Amino (-NH₂), Carboxyl (-COOH) und Amido (-CONH₂) ausgewählt wird oder aus einem Verbindungsmolekül ausgewählt wird, wie einer hydrophilen Aminosäure, wie Prolin (L-Pro), Arginin (L-Arg) und Lysin (L-Lys), Glucono-delta-lacton (GDL) und einem Saccharid; und/oder
die eisphile Gruppe aus Hydroxyl (-OH), Amino (-NH₂), Phenyl (-C₆H₅), Pyrrolidinyl (-C₄H₈N) ausgewählt wird oder aus einem Verbindungsmolekül ausgewählt wird, wie einer eisphilen Aminosäure, wie Glutamin (L-Gln), Threonin (L-Thr) und Asparaginsäure (L-Asn), einem Benzolring (C₆H₆) und Pyrrolidin (C₄H₉N).

## Revendications

1. Procédé de conception moléculaire d'un matériau inhibiteur de formation de glace, comprenant les étapes suivantes :
(1) la construction d'une bibliothèque de structures de molécules composées, dans lequel les molécules composées comprennent un groupe hydrophile et un groupe à affinité avec la glace ;
(2) la simulation et l'évaluation des performances d'étalement de chacune des molécules composées au niveau d'une interface glace-eau en adoptant une simulation de dynamique moléculaire (DM) sur un ordinateur ; et
(3) le filtrage des molécules composées ayant les affinités souhaitées pour la glace et l'eau ;
dans lequel la simulation DM de l'étape (2), la simulation et le calcul sont réalisés sur des interactions entre les molécules composées, les interactions entre les molécules composées et les molécules d'eau, et les interactions entre les molécules composées et les molécules glace-eau ;
dans lequel les interactions comportent la formation d'une liaison hydrogène, d'une interaction de Van der Waals, d'une interaction électrostatique, d'une interaction hydrophobe ou d'une interaction π-π.

2. Procédé de conception moléculaire selon la revendication 1, dans lequel la simulation DM de l'étape (2) est réalisée par GROMACS, AMBER, CHARMM, NAMD ou LAMMPS.

3. Procédé de conception moléculaire selon la revendication 1, dans lequel
dans la simulation DM de l'étape (2), un modèle de molécule d'eau est sélectionné parmi les modèles TIP3P, TIP4P, TIP4P/2005, SPC, TIP3P, TIP5P et SPC/E, de préférence le modèle TIP4P/2005 d'une molécule d'eau ; et/ou dans la simulation DM de l'étape (2), un paramètre de champ de force est fourni par l'un parmi GROMOS, ESFF,
champ de force MM, AMBER, CHARMM, COMPASS, UFF, CVFF et d'autres champs de force ; et/ou
dans la simulation DM de l'étape (2), une température et une pression sont réglées lorsque la simulation et le calcul sont réalisés sur les interactions entre les molécules ; de préférence, la température et la pression sont réglées en utilisant un régulateur de température V-rescale et un régulateur de pression ; et/ou
dans la simulation DM de l'étape (2), une configuration moléculaire des molécules composées est maintenue en sélectionnant un paramètre d'énergie potentielle ; et/ou
dans l'étape (2), des conditions périodiques aux limites sont adoptées pour la direction x, la direction y et la direction z lors de la simulation d'un système de solution aqueuse ; des conditions périodiques aux limites sont adoptées pour la direction x et la direction y lors de la simulation d'un système mixte glace-eau ; et/ou
dans la simulation DM de l'étape (2), une boîte d'eau cubique ou octaédrique est sélectionnée ; et/ou
la chaîne principale des molécules composées est une chaîne carbonée ou une structure de chaîne peptidique ; et/ou
le groupe hydrophile est un groupe fonctionnel capable de former une interaction non covalente avec une molécule d'eau ; et/ou
le groupe à affinité avec la glace est un groupe fonctionnel capable de former une interaction non covalente avec la glace ; et/ou
le matériau inhibiteur de formation de glace est formé par liaison covalente d'un bloc comprenant un groupe hydrophile à un bloc comprenant un groupe à affinité avec la glace, ou est formé par liaison ionique d'une molécule comprenant un groupe hydrophile à une molécule comprenant un groupe à affinité avec la glace.

4. Procédé de conception moléculaire selon la revendication 1, dans lequel
dans la simulation DM de l'étape (2), une boîte d'eau cubique de dimensions 3,9 × 3,6 × 1,0 nm³ est sélectionnée ; et/ou
le groupe hydrophile est un groupe fonctionnel capable de former une liaison hydrogène, une interaction de Van der Waals, une interaction électrostatique, une interaction hydrophobe ou une interaction π-π avec l'eau ; et/ou
le groupe à affinité avec la glace est un groupe fonctionnel capable de former une liaison hydrogène, une interaction de Van der Waals, une interaction électrostatique, une interaction hydrophobe ou une interaction π-π avec la glace.

5. Procédé de conception moléculaire selon la revendication 4, dans lequel
le groupe hydrophile est sélectionné parmi au moins l'un de hydroxyle (-OH), amino (-NH₂), carboxyle (-COOH) et amido (-CONH₂), ou à partir d'une molécule composée, telle qu'un acide aminé hydrophile comme la proline (L-Pro), l'arginine (L-Arg) et la lysine (L-Lys), la glucono delta-lactone (GDL) et un saccharide ; et/ou
le groupe à affinité avec la glace est sélectionné parmi hydroxyle (-OH), amino (-NH₂), phényle (-C₆H₅), pyrrolidinyle (-C₄H₈N) ou à partir d'une molécule composée, telle qu'un acide aminé à affinité avec la glace comme la glutamine (L-Gln), la thréonine (L-Thr) et l'acide aspartique (L-Asn), un cycle benzène (C₆H₆) et la pyrrolidine (C₄H₉N).
